(19) Europäisches Patentamt
European Patent Office
Office européen des brevets

(11) Publication number: 0 390 602 A1

# (12) EUROPEAN PATENT APPLICATION

(21) Application number: 90303470.0

(22) Date of filing: 30.03.90

(51) Int. Cl.[5]: C07K 7/06, C07K 5/04, A61K 37/02

(30) Priority: 30.03.89 JP 80398/89
27.10.89 JP 280590/89
21.12.89 JP 333241/89

(43) Date of publication of application:
03.10.90 Bulletin 90/40

(84) Designated Contracting States:
AT BE CH DE DK ES FR GB GR IT LI LU NL SE

(71) Applicant: SUMITOMO PHARMACEUTICALS COMPANY, LIMITED
2-8, Doshomachi 2-chome, Chuo-ku
Osaka-shi Osaka 541(JP)

Applicant: Ojika, Kosei
18 Miyajinki Kiori Miwacho
Ama-gun, Aichi-ken(JP)

Applicant: Yamamoto, Masahiko
21 Shirakabe-4-chome, Higashi-ku
Nagoya-shi(JP)

(72) Inventor: Ojika, Kosei
18 Miyajinki, Kiori, Miwacho
Ama-gun, Aichi-ken(JP)
Inventor: Ono, Keiichi
10-4, Momoyamadai 3-chome
Sakai-shi(JP)
Inventor: Ikeda, Yoshiharu
4-1-206, Ryodocho
Nishinomiya-shi(JP)
Inventor: Ueki, Yasuyuki
4-2-302 Ryodocho
Nishinomiya-shi(JP)
Inventor: Irie, Tsunemasa
6-12 Mukoyama 2-chome
Takarazuka-shi(JP)
Inventor: Fukushima, Nobuyuki
11-7-406 Sonehigashinocho 2-chome
Toyonaka-shi(JP)

(74) Representative: Cresswell, Thomas Anthony et al
J.A. Kemp & Co. 14 South Square Gray's Inn
London WC1R 5EU(GB)

## (54) Neurotrophic peptides.

(57) A novel neurotrophic peptide of sequence
Ala-Ala-Asp-Ile-Ser-Gln-Trp-Ala-Gly-Pro-Leu
which exhibits biological activity enhancing acetylcholine synthesis in cholinergic neurons is isolated and purified from a water soluble fraction of hippocampal tissue in mammals. A derivative of the peptide having the same biological activity may be chemically synthesized using a conventional solid or liquid phase method. The peptide and derivative thereof are useful for the treatment of neurological degenerative disorders and dimentia.

EP 0 390 602 A1

# NEUROTROPIC PEPTIDES

## BACKGROUND OF THE INVENTION

### Field of the Invention

The present invention relates to novel neurotrophic peptides which are derived from hippocampal tissue in mammals and exhibit a biological activity in enhancing synthesis of acetylcholine in cholinergic neurons, and to their derivatives.

More specifically, the present invention relates to novel neurotrophic peptides which are derived from brain hippocampal tissue in mammals and exhibit various physiological activities in formation and maintenance of cholinergic neurons and derivatives of which also exhibit such activities, and to pharmaceutical compositions for the treatment of neurological degenerative disorders and dementia comprising the peptides or their derivatives as active ingredients.

### Related Art Statement

Neurotrophic factors are generally supplied from target organ in neuro-terminal, glia cell or blood. The factors are known to have biological activities for survival and maintenance of neuronal cell, acceleration of neurite elongation, and for induction of an enzyme for synthesizing neurotransmitters. Especially, in the formation and maintenance of neuronal network with long distance projection, the supplement of neurotrophic factor from target cell is considered to be indispensable. The relation between neuronal degeneration or denervation and neurological diseases has been made clearer. As a typical example, it is suggested that the degeneration/denervation of cholinergic neuron from basal forebrain to hippocampal neocortex is associated with Alzheimer type dementia. There are also suggested the relation between the degeneration/denervation of dopaminergic neuron from substantia nigra to striatum and Parkinson's disease, and the relation between the degeneration/denervation of spinal motor neuron and amyotrophic lateral sclerosis (ALS). These degeneration/denervation, i.e., the cause of these diseases is supposed to be due to the decrease or deficiency in the supplement of neurotrophic factor from target cell, so that there is suggested a possibility for the treatment of neurological degenerative disorders by the supplement of neurotrophic factors [Appel. S.H., Annals of Neurology, 10, 499 (1981)].

Many reports have shown evidence of the existence of neurotrophic factors in mammals. Hitherto, only two neurotrophic factors, nerve growth factor (NGF) and brain-derived neurotrophic factor (BDNF), however, have been isolated and analysed for the primary structures. NGF is composed of three different subunits $\alpha$, $\beta$ and $\gamma$ with the ratio of $\alpha_2\beta\gamma_2$. It is reported that only $\beta$-subunit exhibits the biological activity [Varon, S. et al., Biochemistry, 7, 1296 (1968)]. The primary structure of mouse $\beta$-NGF was determined by Angeletti & Bradshow in 1971 to reveal that $\beta$-HGF is a protein composed of 118 amino acids [molecular weight (Mw); 13,259)], [Angeletti & Bradshow, Proc. Natl. Acad. Sci. USA, 68, 2417 (1971)]. NGF is involved in sympathetic neuronal and sensory neuronal systems at peripheral neuronal systems, and also recognized to be involved in cholinergic neuronal system at the central neuronal systems [Korsching, S. et al., Neuroscience Letters, 54, 201, (1985); Korsching, S. et al., EMBO Journal, 4, 1389, (1985)].

BDNF was purified by Thoenen et al. from hog brain as a neurotrophic factor for survival of sensory neurons in embryonal dosal root ganglion, and the primary structure of BDNF was recently reported by Barde et al. to show that hog BDNF is a protein having 119 amino acids in total [Mw; 13,511) and having very high homology with NGF [Barde, Y.A. et al., EMBO J., 1, 549 (1982); Leiblock J. et al., Nature, 341, 149 (1989)].

Furthermore, a ciliary neurotrophic factor (CNTF) was isolated from chick embryonal eyes as a neurotrophic factor, which had an activity for survival of parasympathetic ganglion (ciliary ganglion) cell [Manthorpe, M. et al., Journal of Neurochemistry, 34, 69 (1980); Manthorpe, M. et al., Journal of Neuroscience Research, 8, 233 (1982); Manthorpe, M. et al., Federation Proceedings, 44, 2753 (1985)].

The primary structure of rat CNTF was recently reported by Stöckli et al., [Stöckli et al., Nature, 342, 920 (1989)] and that of rabbit CNTF was determined by Leu-Fen H. Lin et al. [Science 24, 1023 (1989)]. Rat CNTF is a protein having 200 amino acids in total [Mw; 12800].

Moreover, in addition to the three neurotrophic factors as described above, other several neurotrophic factors have been reported, although they have not yet been highly purified. Some neurotrophic factors that

are derived from hippocampus or act on hippocampus or medial septum nucleous have been reported as follows:

(1) Neurotrophic factor partially purified from hippocampal tissue of neonatal rat (1-2 weeks old) [Ojika, K. et al., Proc. Natl. Acad. Sci. USA, 81, 2567 (1984); Appel. S.H., Japanese Patent KOKAI (Laid-Open) No. 58-154514; Ojika, K., Japanese Journal of Psycopharmacology, 7, 447 (1987)]:
The water soluble factor exists fairly specifically in hippocampal tissue, and enhances the growth of cultured medial septum nucleous cholinergic neuron. The results of partial purification reveal that this factor is a polypeptide having a molecular weight of about 900 daltons. The factor is considered to be different from NGF, because its activity is not inhibited by the addition of anti-NGF antibody [Böstwick, J.H. et al., Brain Research, 422, 92 (1987)].

(2) Neurotrophic factor derived from the conditioned medium of neonatal rat brain astrocyte [Muller, H.W. et al., Proc. Natl. Acad. Sci. USA, 81, 1248 (1984)]:
This factor is considered from elution position of gel filtration to have a molecular weight of about 500 and to be non-protein factor because of the fact that its activity does not still disappear after the treatment with trypsin or pronase.

(3) Neurotrophic factor partially purified from hippocampal extract of rats whose fimbria fornix have been lesioned before 14 days [Yoshida, K., Keiou Igaku, 65, (1), 45, (1988)]:
Three factors having molecular weights of 10,000 to 20,000, 30,000 to 40,000 and 50,000 to 60,000, respectively, as measured by gel filtration have been reported.

(4) Neurotrophic factor derived from rat hippocampal tissue [Heacock, A.M. et al., Brain Research, 363, 299, (1986)]:
This factor exhibits an activity for survival of chick ciliary ganglion cells, and is an acidic protein having a molecular weight of 10,000 or more.

As mentioned above, these hippocampus-derived factors or factors which act on hippocampus or medial septum nucleous have been reported as protein or non-protein factors; however, none of them has been isolated and purified or further determined for their primary structures.

Moreover, NGF and BDNF, already determined for their primary structures, are both proteinacious factors having high molecular weights of 13,259 and 13,511, respectively; CNTF, isolated and characterized biochemically, is also a protein having a high molecular weight of 20,400. In the case where such factors are used as agents for the treatment of neurological degenerative disorders, the factors are likely to meet difficulties in their bioavailability or industrial production, etc.

Therefore, it is desired to develop a highly purified peptidic neurotrophic factor having a lower molecular weight and its derivatives having a similar pharmacological activity.

## SUMMARY OF THE INVENTION

One object of the present invention is to provide novel neurotrophic peptides and derivatives of which have an activity for enhancing acetylcholine synthesis in cholinergic neurons.

Another object of the present invention is to provide methods for producing the novel neurotrophic peptides and their derivatives.

Still another object of the present invention is to provide pharmaceutical compositions for the treatment of neurodegradative disorders and dementia.

Accordingly, one aspect of the present invention is directed to a novel neurotrophic peptide comprising the whole or partial sequence of an amino acid sequence of formula (1):

Ala-Ala-Asp-Ile-Ser-Gln-Trp-Ala-Gly-Pro-Leu (1)

$NH_2$- and/or COOH-terminal-modified derivative of the peptide, or pharmaceutically acceptable salt thereof.

Another aspect of the present invention is directed to a method for preparing the peptide, derivative thereof or salt thereof, which comprises the steps of:

(a) subjecting an extract solution from hippocampal tissue in mammals to reverse phase liquid chromatography, and

(b) collecting the desired product; or which comprises the steps of chemical syntheses.

Still another aspect of the present invention is directed to a pharmaceutical composition for the treatment of neurological degenerative disorders and dementia which comprises the peptide, derivative thereof or salt thereof as an active ingredient.

## BRIEF DESCRPTION OF THE DRAWINGS

Fig. 1 shows the elution pattern of partially purified "HCNP" on reverse phase resin, RP-304 column (4.6 $\phi$ x 250 mm). Vertical line in Fig. 1 shows the concentration of peptide monitored at A220 nm. Horizontal line in Fig. 1 shows the retention time.

Fig. 2 shows the elution pattern of highly purified "HCNP" on Toso's reverse phase resin, TSK-120T column (4.0 $\phi$ x 250 mm).

DETAILED DESCRIPTION OF THE INVENTION

We have investigated to solve the problems as mentioned above and succeeded in the isolation, purification and amino acid-sequencing of a novel neurotrophic peptide originated in hippocampal tissue. This neurotrophic peptide may be abbreviated as HCNP (Hippocampal Cholinergic Neurotrophic Peptide) hereinafter; in the case that the peptide specifically indicates the natural type peptide isolated and purified from extract of hippocampal tissue, an abbreviation "HCNP" may be used.

After our successive investigations, we have found a surprising fact that partial fragments of HCNP, derivatives of HCNP or the fragments and peptides modified in the amino- and/or carboxy-terminal also show a similar activity to that of "HCNP" derived from the extract of hippocampal tissue, and we have thus completed the present invention.

The neurotrophic peptides of the present invention as described herein means a peptidic neurotrophic factor, and shows physiological and biological characteristics of the following (a) to (c):

(a) a peptide in pure form derived from a water soluble fraction of hippocampal tissue;

(b) a molecular weight of 700 to 1,400, as determined by gel filtration with Bio Gel P-2; and

(c) a biological activity for enhancing acetylcholine synthesis in cholinergic neurons.

Alternatively, the neurotrophic peptide and the derivatives each indicate a peptide comprising the whole or partial sequence of an amino acid sequence of the following formula (I):

Ala-Ala-Asp-Ile-Ser-Gln-Trp-Ala-Gly-Pro-Leu (I)

$NH_2$- and/or COOH-terminal-modified derivative of the peptide, or pharmaceutically acceptable salt thereof.

Furthermore, the present invention provides a method for preparing the novel neurotrophic peptide and derivative thereof as mentioned above, and a pharmaceutical composition for the treatment of neuronal degenerative disorder and dementia which comprises the peptide, derivative thereof or salt thereof as an active ingredient.

More detailed explanations as for the neurotrophic peptides and their derivatives of the present invention are give below.

(1) Molecular weight:

A molecular weight of "HCNP" is 700 to 1,400, as determined by gel filtration with Bio Gel P-2 column. As markers, ribonuclease A (Mw, 13,700), vitamin $B_{12}$ (Mw, 1,355), $\beta$-nicotinamide adenine dinucleotide (NAD) (Mw, 633), Z-Glu-Tyr (Mw, 444) and Trp (Mw, 204) were used. Elution buffer used was 0.05 M acetic acid.

(2) Amino acid composition:

Amino acid composition of "HCNP" was determined by hydrolysis with phenol containing constant boiling point-HCl at 110 °C for 20 hours. The amino acid composition as calculated with Leu as a standard amino acid is shown below:

Asx: 0.9, Ser: 1.4, Glx: 1.3, Pro: 1.0 Gly: 1.3, Ala: 2.5, Ile: 0.9, Leu: 1.0, Trp: 0.4

(3) $NH_2$-Terminal amino acid:

"HCNP" was Edman-degraded by a protein sequencer (Applied Biosystems, type 477), then the products, PTH (phenylthiohydantoin)-amino acid derivatives, were analysed by PTH analyzer (Applied Biosystems, type 120A). No amino acid was detected in cycles 1 to 10. The $NH_2$-terminal amino acid of the peptide is suspected to be blocked.

$NH_2$-Terminal amino acid of "HCNP" was removed with acylamino acid-releasing enzyme, and the resulting acylamino acid was collected. Alanine was detected by amino acid analysis after hydrolysis of the

acylamino acid. Thus, the $NH_2$-terminal amino acid was identified as Ala.

(4) Amino acid sequence:

After deletion of the blocked $NH_2$-terminal amino acid of "HCNP" with the treatment of acylamino acid-releasing enzyme, the remaining peptide was collected by reverse phase liquid chromatography, and then subjected to analyses by protein sequencer (Applied Biosystems, type 477) and PTH analyzer (Applied Biosystems, type 120A) to be identified as follows:
Ala-Asp-Ile-Ser-Gln-Trp-Ala-Gly-Pro-Leu.

From the results of the above (3) and (4), the amino acid sequence of "HCNP" was identified as follows:

| 1 | 2 | 3 | 4 | 5 | 6 | 7 | 8 | 9 | 10 | 11 |
|---|---|---|---|---|---|---|---|---|---|---|
| Ala- | Ala- | Asp- | Ile- | Ser- | Gln- | Trp- | Ala- | Gly- | Pro- | Leu. |

(5) COOH-Terminal structure:

"HCNP" was subjected to fragmentation with chymotrypsin, and the resulting COOH-terminal peptide was isolated by reverse phase high performance liquid chromatography, then subjected to amino acid-sequencing to be identified as Ala-Gly-Pro-Leu.

On the other hand, Ala-Gly-Pro-Leu-OH and Ala-Gly-Pro-Leu-$NH_2$ were synthesized by a conventional peptide synthetic method, analyzed by high performance liquid chromatography on reverse phase resin packing column, TSK 120T column, then compared in the retention time with the COOH-terminal peptide. The results reveal that the COOH-terminal peptide is coincident with Ala-Gly-Pro-Leu-OH. Accordingly, the COOH-terminal amino acid at "HCNP" was identified as Leu-OH.

(6) $NH_2$-Terminal structure:

The acylalanine obtained in the above (3) together with various acyl derivatives of amino acids were analysed by HPLC method using reverse phase resin, TSK-120T column, so that the acylalanine was coincident with acetylalanine when compared in the retention times. Thus, the $NH_2$-terminal amino acid was identified as acetylated Ala. A peptide of the following formula:
Acetyl-Ala-Ala-Asp-Ile-Ser-Gln-Trp-Ala-Gly-Pro-Leu
was prepared by a conventional peptide synthesis method, analysed by HPLC on reverse phase resin, TSK-120T column, then compared in the retention time with "HCNP". Because the retention time of "HCNP" was coincident with that of the synthesized peptide, the primary structure of "HCNP" was identified as follows:
Acetyl-Ala-Ala-Asp-Ile-Ser-Gln-Trp-Ala-Gly-Pro-Leu.

The neurotrophic peptide of the present invention, however, also encompasses a free type peptide with free Ala at $NH_2$-terminal. Hereinafter, where it is necessary to show the difference in $NH_2$-terminal structure more clearly, a peptide blocked by acetyl group is referred to as Ac-HCNP and an unblocked free peptide is referred to as f-HCNP.

The neurotrophic peptide derivative of the present invention is used to mean a peptide derivative in which the structure of the parent neurotrophic peptide (HCNP) is modified or converted by three methods for derivation, i.e., fragmentation, modification of the $NH_2$-terminal and modification of the COOH-terminal, singly or in combination.

In more detail, the neurotrophic peptide derivative of the present invention is used to mean a straight chain peptide derivative represented by the following general formula (II):
X-Z-Y (II)
wherein X represents H, various acyl groups or various sulfonyl groups, etc.

Representative examples of X include H;

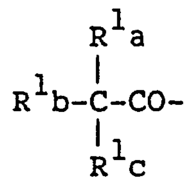

wherein $R^1a$ represents H, an unsubstituted or substituted alkyl group, amino, mono- or di-$C_1$-$C_4$ alkylamino, hydroxy, $-CO_2H$, guanidino, $C_1$-$C_4$ alkylguanidino, aryl, $C_1$-$C_4$ alkoxy, halo, $N(R^2)_3A$ (wherein $R^2$ is $C_1$-$C_4$ alkyl and A represents a counter ion selected from the group consisting of monovalent anions), $-CONR^3R^4$ (wherein each of $R^3$ and $R^4$ independently represents H or $C_1$-$C_4$ alkyl) or a heterocyclic group; $R^1b$ represents H, an unsubstituted or substituted alkyl group or halo; and $R^1c$ represents H, $C_1$-$C_4$ alkyl or halo);

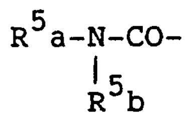

wherein $R^5a$ represents H, $C_1$-$C_4$ alkyl or aryl, and $R^5b$ represents H or $C_1$-$C_4$ alkyl;
$R^6$-O-CO-
wherein $R^6$ represents $C_1$-$C_4$ alkyl or aryl;
$R^7$-CO-
where $R^7$ is H, aryl or a heterocyclic group;
$R^8$-$SO_2$-
wherein $R^8$ is an unsubstituted or substituted alkyl group or aryl; and the like.

Y represents OH, or Y is combined with the carbonyl group at the COOH-terminal of Z to form various amido groups or various ester groups, etc. Representative examples of Y include:

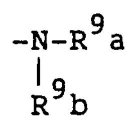

wherein $R^9a$ is H, an unsubstituted or substituted alkyl group, hydroxy, aryl or a heterocyclic group; and $R^9b$ is H or an unsubstituted or substituted alkyl;
-O-$R^{10}$
wherein $R^{1°}$ is an unsubstituted or substituted alkyl group, aryl or a heterocyclic group;

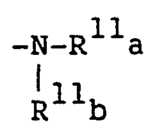

wherein $R^{11}a$ and $R^{11}b$ are combined together to form a nitrogen-containing saturated heterocyclic group; and the like.

Z is a partial amino acid sequence of HCNP composed of at least consecutive two residues, generally at least 3 consecutive residues, or the whole amino acid sequence of HCNP, which can be obtained by any one of fragmentation for decreasing amino acid residues of the amino acid sequence (Ala-Ala-Asp-Ile-Ser-Gln-Trp-Ala-Gly-Pro-Leu) of HCNP from the $NH_2$-terminal, fragmentation for decreasing amino acid residues from the COOH-terminal thereof and fragmentation for decreasing amino acid residues from both the COOH and $NH_2$-terminals. Preferably, Z is an amino acid sequence represented by general formula:
$Z^1$-Trp-$Z^2$
wherein $Z^1$ represents Ala-Ala-Asp-Ile-Ser-Gln, Ala-Asp-Ile-Ser-Gln, Asp-Ile-Ser-Gln, Ile-Ser-Gln, Ser-Gln, Gln or a single bond; $Z^2$ represents Ala-Gly-Pro-Leu, Ala-Gly-Pro, Ala-Gly, Ala or a single bond; provided that when $Z^1$ represents a single bond, $Z^2$ cannot be a single bond.

Representative examples of Z which are more preferred include:
Ala-Ala-Asp-Ile-Ser-Gln-Trp-Ala-Gly-Pro-Leu;
Ala-Ala-Asp-Ile-Ser-Gln-Trp-Ala-Gly-Pro;
Ala-Ala-Asp-Ile-Ser-Gln-Trp-Ala-Gly;
Ala-Ala-Asp-Ile-Ser-Gln-Trp-Ala;
Ala-Ala-Asp-Ile-Ser-Gln-Trp;
Ala-Asp-Ile-Ser-Gln-Trp-Ala-Gly-Pro-Leu;
Ile-Ser-Gln-Trp-Ala-Gly-Pro-Leu;
Gln-Trp-Ala-Gly-Pro-Leu;
Ala-Asp-Ile-Ser-Gln-Trp-Ala-Gly-Pro;
Ala-Asp-Ile-Ser-Gln-Trp-Ala;
Ala-Asp-Ile-Ser-Gln-Trp;
Asp-Ile-Ser-Gln-Trp-Ala-Gly; and,
Ile-Ser-Gln-Trp-Ala.

Where the abbreviation HCNP is used to simply refer to a structure of derivative, the abbreviation HCNP means:
H-Ala-Ala-Asp-Ile-Ser-Gln-Trp-Ala-Gly-Pro-Leu-OH.

The term "alkyl" used in the present invention means a branched and straight saturated aliphatic hydrocarbon group having a specific number of carbon atoms. For example, $C_1$-$C_4$ alkyl means methyl, ethyl, propyl, butyl, isopropyl, isobutyl, t-butyl, etc.

The term "alkoxy" means an alkyl group having a specific number of carbon atoms which is bound via an oxygen atom. For example, $C_1$-$C_4$ alkoxy means methoxy, ethoxy, propoxy, butoxy, etc.

The term "$C_1$-$C_4$ alkylguanidino" means a guanidino group in which the guanidino nitrogen atom is alkylated by one or two $C_1$-$C_4$ alkyl groups.

The term "halo" means fluoro, chloro, bromo and iodo.

The term "counter ion" means monovalent anion such as chloride, bromide, acetate, perchlorate, benzoate, maleate, benzenesulfonate, tartarate, hemitartarate, etc.

The term "aryl" is used to mean phenyl, naphthyl or anthryl, etc. which may optionally be substituted with 1 to 3 groups independently selected from the group consisting of: $C_1$-$C_8$ alkyl, amino, mono- or di-$C_1$-$C_4$ alkylamino, amino-$C_1$-$C_8$ alkyl, mono- or di-$C_1$-$C_4$ alkylamino-$C_1$-$C_8$ alkyl, guanidino, $C_1$-$C_4$ alkylguanidino, guanidino-$C_1$-$C_8$ alkyl, $C_1$-$C_4$ alkylguanidino-$C_1$-$C_8$ alkyl, hydroxyl, hydroxyl-$C_1$-$C_8$ alkyl, $C_1$-$C_4$ alkoxy, $-CO_2H$, carboxy- $C_1$-$C_8$ alkyl, halo, $NO_2$, $CF_3$ and $-CONR^3R^4$ [$R^3$ and $R^4$ have the same significances as described above] and the like.

The term "heterocyclic group" is used to mean a saturated or unsaturated 3- to 8-membered monocyclic or 9-to 10-membered fused heterocyclic group. The heterocyclic group may be composed of carbon atoms and 1 to 3 hetero atoms selected from the group consisting of N, O and S. The nitrogen and sulfur hetero atoms may optionally be oxidized; or the nitrogen hetero atom may optionally be quaternized. The binding site may be on any of the carbon atoms but with respect to $R^1a$, in the case of a hetero ring containing at least one nitrogen atom, the nitrogen atom can be the binding site. Examples of such saturated heterocyclic group include pyrrolidinyl, piperidyl, piperidino, homopiperidyl, heptamethyleneiminyl, piperazinyl, homopiperazinyl, morpholinyl, morpholino, thioranyl, thiomorpholinyl, thiomorpholinylsulfoxide, thiomorpholinylsulfone, tetrahydrofuryl, etc. Such saturated heterocyclic moieties may also be optionally substituted with 1 or 2 groups independently selected from the group consisting of: hydroxy, carboxyl, carboxyl-$C_1$-$C_8$ alkyl, aryl, aryl-$C_1$-$C_4$ alkyl, $C_1$-$C_4$ alkyl, amino, mono-or di-$C_1$-$C_4$ alkylamino, amino-$C_1$-$C_8$ alkyl, mono- or di-$C_1$-$C_4$ alkylamino-$C_1$-$C_8$ alkyl, hydroxy-$C_1$-$C_4$ alkyl, guanidino, $C_1$-$C_4$ alkylguanidino, guanidino-$C_1$-$C_8$ alkyl, $C_1$-$C_4$ alkylguanidino-$C_1$-$C_8$ alkyl, $-N(R^2)_3A$ (wherein $R^2$ and A have the same significances as described above) and $N(R^2)_3$ A-substituted $C_1$-$C_8$ alkyl (wherein $R^2$ and A have the same significances as described above) and the like. Examples of such unsaturated heterocyclic groups include pyrrolyl, pyridyl, pyrazinyl, imidazolyl, pyrazolyl, furyl, oxazolyl, thienyl, thiazolyl, indolyl, quinolyl, isoquinolyl, etc. Such unsaturated heterocyclic group may optionally be substituted with a group selected from the group consisting of $CF_3$, $C_1$-$C_4$ alkyl, $C_1$-$C_4$ alkoxy, halo, etc. The term "nitrogen-containing saturated heterocyclic group" means a saturated 3- to 8-membered monocyclic nitrogen-containing heterocyclic group, which is composed of at least one nitrogen atom and carbon atoms and which may further contain one hetero atom selected from the group consisting of O and S and which binding site is on the nitrogen atom. The nitrogen and sulfur hetero atoms may optionally be oxidized; or the nitrogen atom may also be quaternized. Examples of such nitrogen-containing saturated heterocyclic group include pyrrolidinyl, piperidino, homopiperidino, heptamethyleneiminyl, piperazinyl, homopiperazinyl, morpholino, thiomorpholino, thiomorpholinosulfoxide, thiomorpholinosulfone, etc. Such nitrogen-containing

saturated heterocyclic groups may also be optionally substituted with 1 or 2 groups independently selected from the group consisting of: hydroxy, carboxyl, carboxyl-$C_1$-$C_8$ alkyl, aryl, aryl-$C_1$-$C_4$ alkyl, $C_1$-$C_4$ alkyl, amino, mono- or di-$C_1$-$C_4$ alkylamino, amino-$C_1$-$C_8$ alkyl, mono- or di-$C_1$-$C_4$ alkylamino-$C_1$-$C_8$ alkyl, hydroxy-$C_1$-$C_4$ alkyl, guanidino, $C_1$-$C_4$ alkylguanidino, guanidino-$C_1$-$C_8$ alkyl, $C_1$-$C_4$ alkylguanidino-$C_1$-$C_8$ alkyl, $-N(R^2)_3A$ (wherein $R^2$ and A have the same significances as described above) and $N(R^2)_3A$-substituted $C_1$-$C_8$ alkyl (wherein $R^2$ and A have the same significances as described above) and the like.

The term "unsubstituted or substituted alkyl" means an unsubstituted branched and straight saturated aliphatic hydrocarbon group having 1 to 16 carbon atoms, i.e., $C_1$-$C_{16}$ alkyl, which may optionally be substituted with a group selected from the group consisting of: amino, mono- or di-$C_1$-$C_4$ alkylamino, hydroxy, $-CO_2H$, guanidino, $C_1$-$C_4$ alkylguanidino, aryl, $C_1$-$C_4$ alkoxy, halo, $-N(R^2)_3A$ (wherein $R^2$ and A have the same significances as described above), $-CONR^3R^4$ (wherein $R^3$ and $R^4$ have the same significances as described above) and a heterocyclic group (in the case of a heterocyclic group containing at least one nitrogen atom, the nitrogen atom can be the binding site).

In the specification, amino acids, protective groups, active groups, solvents and the like are sometimes referred to by their abbreviations based on IUPAC-IUB and abbreviations conventionally used in the art. These abbreviations are shown below.

| Abbreviations | Name |
|---|---|
| Ala | L-Alanine |
| Asp | L-Aspartic acid |
| Gly | Glycine |
| Ile | L-Isoleucine |
| Leu | L-Leucine |
| Pro | L-Proline |
| Gln | L-Glutamine |
| Ser | L-Serine |
| Trp | L-Tryptophan |
| Asx | L-Asparagine or L-Aspartic acid |
| Glx | L-Glutamine or L-Glutamic acid |

Unless otherwise indicated, the amino acid residues given without the prefix "L" in the specification correspond to the naturally occurring absolute configuration L.

Other abbreviations are shown below.

| Abbreviations | Name |
|---|---|
| Ac | Acetyl |
| For | Formyl |
| Boc | t-Butyloxycarbonyl |
| $(Boc)_2O$ | di-t-Butyldicarbonate |
| Z | Benzyloxycarbonyl |
| Z-Cl | Benzyloxycarbonyl chloride |
| Tos | p-Toluenesulfonyl |
| Tos-Cl | p-Toluenesulfonyl chloride |
| OcHex | Cyclohexyl ester |
| Bzl | Benzyl |
| DCC | Dicyclohexylcarbodiimide |
| EDC.HCl | 1-Ethyl-3-(3-dimethylaminopropyl)-carbodiimide hydrochloride |
| DMF | Dimethylformamide |
| NMP | N-Methyl-2-pyrrolidinone |
| THF | Tetrahydrofuran |
| AcOEt | Ethyl acetate |
| TFA | Trifluoroacetic acid |
| TEA | Triethylamine |
| CHA | Cyclohexylamine |
| MBHA | 4-Methylbenzhydrylamine |
| HOBt | 1-Hydroxybenzotriazole |
| HONp | p-Nitrophenol |
| PTC | Phenylthiocarbamyl |
| ACh | Acetylcholine |
| FCS | Fetal Calf Serum |

The pharmaceutically acceptable salts of the neurotrophic peptide and derivatives of the present invention include conventional non-toxic salts of these peptides and quaternary salts thereof. These salts may be formed from inorganic or organic acids or bases. Examples of such acid addition salts are salts of acetic acid, butyric acid, citric acid, lactic acid, tartaric acid, oxalic acid, maleic acid, succinic acid, fumaric acid, hydrochloric acid, hydrobromic acid and sulfuric acid. Salts of bases are ammonium salts, alkali metal salts such as sodium and potassium salts, alkaline earth metal salts such as calcium and magnesium salts, salts with amino acids such as arginine, lysine, etc. Such salts can be readily produced by known methods. For example, in the case of acetates, the acetates can be prepared by dissolving the neurotrophic peptide or its derivative in water and adding a necessary amount of acetic acid to the solution.

For preparation of "HCNP" of the present invention, for example, the following method can be illustratively shown.

Methods for extraction, purification and isolation of "HCNP" from hippocampal tissue:

As the source of the peptide, brain of any other species of mammals as well as human brain may be used. Such examples include mouse, rat, dog, calf, horse, goat, sheep, rabbit, hog, etc. A typical example is hippocampal tissue in rat brain.

Preferably, hippocampal tissue is taken out from brain of neonatal rat of 1 to 2 week old, then frozen to be stocked until use, preferably at below -70 °C.

The extraction from hippocampal tissue is carried out under cooled condition, preferably at 0 °C to 5 °C. To hippocampal tissue, 2 to 10 volumes of neutral pH buffer, preferably 6 to 7 volumes of phosphate buffer of pH 7.2 is added, followed by the homogenization with glass teflon homogenizer. After centrifugation, preferably at 100,000 G. at 4 °C for 2 hours, the resulting debris is discarded.

To the obtained supernatant an acid, preferably acetic acid at final concentration of 2 M is added, to give an acidified solution and the resulting debris is discarded by centrifugation.

The supernatant thus obtained can be purified by conventional methods. For example, gel filtration chromatography, ultrafiltration, dialysis and salting-out may be used for alone or combined purification. Purification is preferably carried out under cooled condition, preferably at 0 °C to 5 °C.

Preferably, the obtained supernatant is subjected to ultrafiltration filter which passes a low molecular weight of below 5,000, for example, YM5 memberane (Amicon), then the collected fraction is freeze-dried.

The powder thus obtained is dissolved in small volume of an acid, preferably 0.05 M acetic acid, then subjected to molecular weight-fractionation by gel filtration chromatography on an appropriate resin, for example, Biogel P-2, Biogel P-6 or Sephadex G-25, preferably Biogel P-2.

The eluate is assayed for an activity on the acetylcholine synthesis in cholinergic neuron by biological assay method using rat medial septum nucleous.

Fractions exhibiting a biological activity are collected and applied to an appropriate reverse phase type resin, preferably Sep.Pak-C18 cartridge (Millipore). After washing the cartridge with an acid, preferably 0.05 M acetic acid, elution is carried out with an appropriate acidic organic solvent, preferably 0.05 M acetic acid-60% acetonitrile.

Collected eluate is freeze-dried to give white powder.

From the white powder, "HCNP" can be isolated, singly or in combination, by chromatographies such as reverse phase liquid chromatography, ion-exchange chromatography and affinity chromatography using an appropriate antibody.

Preferably, the white powder is dissolved in an appropriate acid, preferably 0.1% TFA, then purified by reverse phase liquid chromatography on a suitable reverse phase type resin, for example, $C_1$-$C_{18}$ alkyl- or phenyl-silica gel carrier.

As an elution solvent there may be used acetonitrile, propanol, isopropanol, methanol, ethanol or butanol each containing TFA, acetic acid or phosphoric acid. For example, a solution of the white powder may be fractionated by HPLC on RP 304 reverse phase column (Bio-Rad) and the fractions eluted with acetonitrile at 21 to 26% concentration are collected and further freeze-dried to white powder.

This white powder is dissolved in a suitable acid, preferably 0.05% TFA, fractionated by HPLC on TSK-120T reverse phase column (Toso) to collect the 28% to 30% acetonitrile-fractions, then frozen to dryness. The obtained white powder, "HCNP", is usually stocked in cool and dark place.

For preparing the neurotrophic peptide of the present invention and its derivative, the following method by chemical synthesis may also be used.

Method by chemical synthesis:

According to the method by chemical synthesis, the neurotrophic peptide can be synthesized in a manner similar to methods conventionally used in ordinary peptide chemistry. Such known methods are described in, for example, M. Bodansky and M.A. Ondetti, Peptide Synthesis, published by Interscience Publishing Co., New York, 1966; F.M. Finn and K. Hofmann, the Proteins, volume 2, edited by H. Neurath, R.L. Hill, Academic Press Inc., New York, 1976; Nobuo Izumiya, Pepuchido Gousei, published by Maruzen Co., 1975; Nobuo Izumiya, Pepuchido Gousei no Kiso to Jikken, published by Maruzen Co., 1985; Seikagaku Jikken Kouza, edited by the Japanese Biochemical Society, volume 1, Tanpakushitu no Kagaku IV, chapter II; Haruaki Yajima, Pepuchido Gousei, 1977; etc. That is, the peptide can be synthesized by selecting any of the liquid phase method and the solid phase method, depending upon the structure of the COOH-terminal. In more detail, where peptides contain a partial structure of -COOH or -$CONH_2$ at the COOH-terminal, the peptides can be obtained by any of the liquid phase method and the solid phase method but in other cases, the liquid phase method is rather preferred.

For example, in the case that the peptide is synthesized by the solid phase method, the COOH-terminal amino acid (amino group-protected amino acid) or the COOH-terminal substituent (the substituent having carbonyl group; in the case of containing an amino group, the amino group is protected) is bound to insoluble carrier through the carboxyl group therein. If necessary and desired, after the amino protecting group is then removed, the amino group-protected amino acids are successively coupled, according to the amino acid sequence of the desired peptide, through condensation of the reactive carboxyl groups with the reactive amino groups or with the reactive hydroxy groups. The synthesis is carried out step by step. After synthesis of the whole sequence, if necessary and desired, the $NH_2$-terminal substituent such as acyl or sulfonyl groups described above may be condensed with the whole sequence. Thereafter, the resulting peptide is cleaved from the insoluble carrier and at the same time, the protecting group is removed to obtain the desired peptide.

In the case of synthesis by the liquid phase method, the COOH-terminal amino acid having a free amino group at the terminal (carboxyl group-protected amino acid) or the COOH-terminal substituent (the substituent having free amino or hydroxy group; in the case that a carboxyl group is present, the carboxyl group is protected) is coupled with the amino group-protected amino acid, according to the amino acid

sequence of the desired peptide. Then, after the amino protecting group is removed, the amino group-protected amino acids are successively coupled, through condensation of the reactive amino groups with the reactive carboxyl groups and, if necessary, the $NH_2$-terminal substituent such as acyl or sulfonyl groups described above is finally condensed therewith. Thus, the whole sequence can be synthesized. The whole sequence may also be synthesized by condensation of the peptide fragments synthesized in a similar manner. The protecting group is removed to obtain the desired peptide.

In the methods described above, the reactive functional groups are preferably protected.

Examples of the protecting group of the amino group include benzyloxycarbonyl, t-butyloxycarbonyl, p-methoxybenzyloxycarbonyl, 2-chlorobenzyloxycarbonyl, p-toluenesulfonyl, trifluoroacetyl, phthalyl, formyl, o-nitrophenylsulfenyl, 3-nitro-2-pyridinesulfenyl, diphenylphosphinothioyl, etc. Examples of the protecting group of the carboxyl group include alkyl esters (esters of $C_1$-$C_4$ such as methyl, ethyl, t-butyl, etc.), benzyl ester, p-nitrobenzyl ester, p-methylbenzyl ester, cyclohexyl ester, cyclopentyl ester, etc. The hydroxy group in Ser, etc. may not be necessarily protected but if necessary, can be protected with benzyl, 2,6-dichlorobenzyl, t-butyl, benzyloxycarbonyl, acetyl, etc. The indolyl group in Trp, etc. may be protected, if necessary, with formyl, benzyloxycarbonyl, 2,4-dichlorobenzyloxycarbonyl, etc. The quanidino group may also function to be protected in the state protonated with hydrochloric acid, etc. but, if necessary, may also be protected with p-toluenesulfonyl, nitro, benzyloxycarbonyl, p-methoxybenzenesulfonyl, mesitylene-2-sulfonyl, etc. In the methods described above, peptide bonds can be formed by known methods, for example, the method using condensing agents of carbodiimide type such as dicyclohexylcarbodiimide, 1-ethyl-3-(3-dimethylaminopropyl)carbodiimide, etc.; the symmetrical anhydride method, the mixed anhydride method, the azide method, the activated ester method, the oxidation-reduction method, the diphenylphosphoryl azide method, the method using carbodiimide type condensing agent and additives (1-hydroxybenzotriazole, N-hydroxysuccinimide, etc.).

For removing the protecting group, there are known, for example, the trifluoroacetic acid method, the methanesulfonic acid method, the trifluoromethanesulfonic acid method, the hydrogen fluoride method, the liquid ammonia-sodium method, the catalytic reduction method, the alkali saponification method, etc.

The peptides produced by the present invention can be purified by known methods conventionally used in the art of peptide chemistry, singly or in combination, such as ion exchange chromatography, partition chromatography, gel chromatography, reverse phase liquid chromatography, etc.

Pharmacological activies:

The neurotrophic peptide and its derivative of the present invention can regulate differentiation and maturation of neuronal cells. That is, the neurotrophic peptide and its derivative accelerate the acetylcholine synthesis in the tissue of medial septum nucleous which is rich in cholinergic neuron. The biological activity can be determined by the method of Ojika, K., et al. [Ojika, K., et al., Japanese Journal of Psycopharmacology, 7, 447-451, (1987)].

Application to therapeutic agents:

The neurotrophic peptide and its derivative of the present invention are useful for the treatment of neurological degenerative disorder and dementia. The neurological degenerative disorder is a disease caused by degeneration/denervation of cholinergic neuron and is exemplified by Alzheimer syndrome, Alzheimer type dementia, amyotrophic lateral sclerosis, Parkinsonism-dementia complex, etc.

As dementia, there are Alzheimer type dementia, Parkinson's dementia and cerebro-vascular dementia.

Animal to which the peptides of the present invention are applicable is not limited. The peptides of the present invention can be applied to human beings as well as to other various species of mammals such as mouse, rat, dog, calf, horse, goat, sheep, rabbit, hog, etc.

The peptides of the present invention can be administered to these animals and human by ordinary routes, for example, orally, intramuscularly, intravenously, subcutaneously, intraperitoneally, pernasally and intracerebrally. Dose and time of administration vary depending upon animal species, administration routes, condition of disease, body weight, etc. In human, the peptides can be administered to adult in a daily dose of approximately 1 µg to 1 g, once or in several portions. Examples of pharmaceutical preparations include powders, granulates, granules, tablets, capsules, suppositories, injections, nasal preparations, etc.

The pharmaceutical preparations can be prepared in a conventional manner, using conventional carriers for preparations. That is, in the case of preparing oral preparations, excipients or carriers are added to the

active ingredient and if necessary, binders, disintegrators, lubricants and coloring agents are further added thereto and then prepared into tablets, granules, powders, capsules, etc., by known methods. In the case of preparing injections, pH regulators, buffers, stabilizers, solubilizing agents, etc. are added depending upon necessity and prepared into injections in a conventional manner.

Hereafter the present invention is described in more detail by referring to the examples below but is not deemed to be limited thereto.

Example 1

Purification of "HCNP":

(1) To the hippocampal tissue corresponding to about 300 rats of 2 week old was added 6- to 7-fold volume of ice-cold phosphate buffer (pH 7.2). After homogenizing with a glass teflon homogenizer, centrifugation was performed at 4°C for 2 hours at 100,000 x g. Glacial acetic acid was added to the supernatant in the final concentration of 2 M and the precipitates were centrifuged at 4°C for 2 hours at 100,000 x g. The supernatant was subjected to super ultrafiltration using YM5 filter (cut-off molecular weight, 5000) manufactured by Amicon Co., Ltd. After the filtrate was frozen to dryness, the freeze-dried product was dissolved in 0.05 M acetic acid and the solution was applied to a column (1.6 x 84 cm) packed with Bio Gel P2 gel previously equilibrated with 0.05 M acetic acid. Elution was performed with 0.05 M acetic acid at a flow rate of 16.5 ml/hour and the eluent was fractionated at 1.65 ml/fraction. Using a part of the fraction, absorbance was measured at A280 nm and the biological activity was determined. The fractions showing the activity were collected and applied to Sep.Pak-C18 cartridge manufactured by Millipore Co., Ltd. After washing with 0.05 M acetic acid, the active ingredient was eluted with 0.05 M acetic acid-60% acetonitrile. The eluent was lyophilized to give the crude product containing 6.8 mg of protein.

The biological activity of the crude product was determined. The product showed the effect of enhancing the acetylcholine synthesis activity of cholinergic neuron to 150%, at a concentration of 160 ng/ml.

(2) The crude product, 3.4 mg, obtained in (1) was dissolved in 0.1% TFA and the solution was applied to reverse phase RP-304 column (4.6 $\phi$ x 250 mm) manufactured by Bio-Rad Co., Ltd., previously equilibrated with 0.1% TFA. Using 2 kinds of solvents, A1 (0.1% TFA) and B1 (0.1% TFA-95% acetonitrile), elution was carried out while increasing the acetonitrile concentration from solvent A1 to solvent B1. For the initial 10 minutes, the concentration of B1 was linearly increased from 0% to 10%, then increased to 30% for the following 40 minutes, to 40% for the following 10 minutes, to 60% for the following 10 minutes and then to 100% for the following 3 minutes, whereby elution was performed at a flow rate of 1.0 ml/min. The eluent was monitored at A220 nm and fractionated at 2 ml/fraction (Fig. 1). The procedure of this reverse phase high performance liquid chromatography was repeated twice. Active fraction Nos. 21 through 26 (fractions eluted at the acetonitrile concentration of 21 to 26%) were collected and concentrated in vacuo with a speed back concentrator to obtain white powders. The biological activity of the white powders was determined and as the result, the white powders showed the activity of enhancing the acetylcholine synthesis ability of cholinergic neuron to 150%, at a concentration of $5.3/10^5$ of the total volume in 1 ml of medium.

(3) The active substance obtained in (2) was dissolved in 0.1% TFA and the solution was applied to reverse phase TSK-120T column (4.0 $\phi$ x 250 mm) manufactured by Toso Corporation, which had been previously equilibrated with 0.1% TFA. Using 2 kinds of solvents, A1 and B1, employed in (2), elution was carried out, while increasing the acetonitrile concentration from solvent A1 to solvent B1. For the initial 5 minutes, solvent A1 alone was passed. The concentration of B1 was linearly increased from 0% to 20% for the following 10 minutes, then increased to 30% for the following 25 minutes, to 40% for the following 10 minutes and then to 100% for the following 10 minutes, whereby elution was performed at a flow rate of 1.0 ml/min. The eluent was monitored at A220 nm and fractionated at 1 ml/fraction. Active fraction Nos. 46 and 47 (fractions eluted at the acetonitrile concentration of 28 to 30%) were collected and concentrated in vacuo.

The active fraction was subjected to chromatography on reverse phase TSK-120T column. Using 2 kinds of solvents, A2 (0.05% TFA) and B2 (0.05% TFA-95% acetonitrile), elution was carried out, while increasing the acetonitrile concentration from solvent A2 to solvent B2. For the initial 5 minutes, the concentration of B1 was linearly increased from 0% to 25%, then increased to 35% for the following 30 minutes, to 40% for the following 5 minutes, and then to 100% for the following 5 minutes, whereby elution

was performed at a flow rate of 1.0 ml/min. The eluent was monitored at A220 nm and the respective peaks were manually fractionated. The activity was recovered in the sharp single peak fraction eluted in fraction Nos. 30 and 31 (acetonitrile concentration of 31 to 32%) (Fig. 2). This active fraction was concentrated in vacuo to give 4 µg of "HCNP".

Example 2

Structural analysis of "HCNP"

(1) Composition of amino acid

After "HCNP" was hydrolyzed at 110°C for 20 hours with 0.2 ml of constant boiling point hydrochloric acid containing phenol, its amino acid was analyzed by the PICO-TAG method. The amino acid composition determined using Leu as a standard amino acid was as follows.
Asx: 0.9, Ser: 1.4, Glx: 1.3, Pro: 1.0, Gly: 1.3, Ala: 2.5, Ile: 0.9, Leu: 1.0, Trp: 0.4

(2) $NH_2$-Terminal amino acid

After 0.5 µg of "HCNP" was degraded by the Edman method using Protein Sequencer, Type 477, manufactured by Applied Biosystems Co., Ltd., the produced PTH (phenylthiohydrantoin)-amino acid derivative was analyzed with PTH Analyzer, Type 120, manufactured by Applied Biosystems Co., Ltd. As the result, no amino acid was detected at all in cycles 1 through 10. Accordingly, it is evident that the $NH_2$-terminus of "HCNP" was blocked.

(3) Structural analysis of peptide fragments obtained by fragmentation with chymotrypsin

After 0.8 µg of "HCNP" was dissolved in 0.1 ml of 0.05 M $NH_4HCO_3$ solution, 0.4 µg of chymotrypsin manufactured by Sigma Co., Ltd. was added to the resulting solution followed by incubation at 37°C. Six hours after, 0.4 µg of chymotrypsin was further added to the system followed by incubation at 37°C overnight.

The reaction solution was applied to reverse phase TSK-120T column (4 ϕ x 250 mm) previously equilibrated with 0.05% TFA. Using 2 kinds of solvents A2 (0.05% TFA) and B2 (0.05% TFA-95% acetonitrile), elution was carried out, while increasing the acetonitrile concentration from solvent A2 to solvent B2. For the initial 40 minutes, the concentration of B2 was linearly increased from 0% to 40% and then increased to 100% for the following 15 minutes, whereby elution was performed at a flow rate of 1.0 ml/min. The eluent was monitored at A220 nm and A280 nm. The active substances were fractionated in peak fractions eluted at retention time of 24 minutes and 36 minutes, which are named CH1 and CH2, respectively.

CH1 and CH2 were concentrated in vacuo, respectively and 0.2 ml of constant boiling point hydrochloric acid containing 0.1% thioglycolic acid was added to the residues. After each of the mixtures was hydrolyzed at 110°C for 24 hours, the respective amino acid were analyzed. The amino acid of each fraction was as follows.
Amino acid composition of CH1:
Pro: 1.17, Gly: 0.61, Ala: 0.82, Leu: 1.00
Amino acid composition of CH2:
Asx: 1.05, Ser: 1.18, Glx: 1.25, Gly: 0.50, Ala: 2.00, Ile: 0.83
Trp was obviously detected but it was impossible to quantitatively determine Trp because of a trace amount.

The amino acid sequences of CH1 and CH2 were identified by Protein Sequencer, Type 477 and PTII Analyzer, Type 120A.
Amino acid sequence of CH1:

1 2 3 4

Ala-Gly-Pro-Leu

In CH2, no amino acid was detected at all in cycles 1 through 10. It was thus concluded that the $NH_2$-terminus was blocked.

In order to reveal the structure of the COOH-terminus of CH1, analysis was made on two synthetic peptides:

H-Ala-Gly-Pro-Leu-OH and H-Ala-Gly-Pro-Leu-$NH_2$

under the conditions as described above by reverse phase high performance liquid chromatography. The retention time of CH1 was coincident with that of the former synthetic peptide. It was thus concluded that the COOH-terminal amino acid, Leu of CH1, was in the form of -OH.

CH1 was dissolved in 340 $\mu$l of 100 mM pyridine-acetate buffer (pH 5.5) and 100 pmols of carboxypeptidase Y was added to the solution followed by incubation at 37° C. Small aliguot of samples were sampled with passage of time to perform the amino acid analysis. As the result, the sequence of Ile-Ser-Gln-Trp was identified as the amino acid sequence of the COOH-terminus of CH2.

CH2 was dissolved in 50 $\mu$l of 0.1 M phosphate buffer (pH 7.2) and 0.1 U of acylamino acid-releasing enzyme manufactured by Takara Shuzo Co., Ltd. was added to the solution followed by incubation at 37° C. Five hours after and 8.5 hours after, 0.05 U each of acylamino acid-releasing enzyme was added to the system followed by incubation at 37° C for 22.5 hours in total. The reaction solution was applied to reverse phase TSK-120T column (4 $\phi$ x 250 mm) previously equilibrated with 0.05% TFA. Using 2 kinds of solvents, A2 (0.05% TFA) and B2 (0.05% TFA-95% acetonitrile), elution was carried out, while increasing the acetonitrile concentration from solvent A2 to solvent B2. For the initial 5 minutes, solvent A2 alone was passed and the concentration of B2 was linearly increased from 0% to 40% for the following 40 minutes and then increased to 100% for the following 15 minutes, whereby elution was performed at a flow rate of 1.0 ml/min. The eluent was monitored at A220 nm and A280 nm. The peak fraction eluted with retention time of 37 minutes was fractionated, which is named CH2-AR1. After CH2-AR1 was hydrolyzed at 110° C for 24 hours with constant boiling point hydrochloric acid containing 0.1% thioglycolic acid, its amino acid was analyzed. Because the amount was trace, the amino acid of 35% B2 solution prepared by mixing solvent A2 and solvent B2 used for the fractionation was also analyzed by hydrolyzing the solution in a similar manner and the amino acid composition of CH2-AR1 was corrected thereby. The amino composition of CH2-AR1 was thus determined.

Amino acid composition of CH2-AR1

Asx: 1.09, Ser: 0.86, Glx: 0.97, Ala: 1.00, Ile: 1.18

Trp was obviously detected but it was impossible to quantitatively determine Trp because of a trace amount.

In CH2-AR1, Ala was less by one residue than the amino acid composition of CH2 described above. The $NH_2$-terminal amino acid was thus identified as Ala.

As the result of analysis of CH2-AR1 with Protein Sequencer, Type 477 and PTH Analyzer, Type 120A, it was identified as Ala-Asp-Ile-Ser-Gln-X.

The foregoing results reveal that the amino acid sequence of CH2 was identified as Ala-Ala-Asp-Ile-Ser-Gln-Trp and the $NH_2$-terminal amino acid, Ala, was blocked.

(4) Structural analysis of peptide fragments obtained by fragmentation with acylamino acid-releasing enzyme

About 2 $\mu$g of "HCNP" was dissolved in 50 $\mu$l of 0.1 M phosphate buffer (pH 7.2) and 0.1 U of acylamino acid-releasing enzyme manufactured by Takara Shuzo Co., Ltd. was added to the solution followed by incubation at 37° C. Five hours after and 8.5 hours after, 0.05 U each of acylamino acid-releasing enzyme was added to the system followed by incubation at 37° C for 22.5 hours in total. The reaction solution was applied to reverse phase TSK-120T column (4 $\phi$ x 250 mm) previously equilibrated with 0.05% TFA.

Using 2 kinds of solvents, A2 (0.05% TFA) and B2 (0.05% TFA-95% acetonitrile), elution was carried out, while increasing the acetonitrile concentration from solvent A2 to solvent B2. For the initial 5 minutes, solvent A2 alone was passed and the concentration of B2 was linearly increased from 0% to 40% for the following 40 minutes and then increased to 100% for the following 15 minutes, whereby elution was

performed at a flow rate of 1.0 ml/min. The eluent was monitored at A220 nm and A280 nm and fractionated by 1 ml/fraction. The peak fraction eluted with retention time of 43 minutes was manually fractionated, which is named AR1. After AR1 was hydrolyzed at 110°C for 24 hours with 0.2 ml of constant boiling point hydrochloric acid containing 0.1% thioglycolic acid its amino acid was analyzed.

Amino acid composition of AR1:

Asx: 0.89, Ser: 0.87, Glx: 1.01, Pro: 0.92, Gly: 1.03, Ala: 1.86, Ile: 0.95, Leu: 1.00, Trp: 0.63

The amino acid sequence of AR1 was identified using Protein Sequencer, Type 477 and PTH Analyzer, Type 120, manufactured by Applied Biosystems Co., Ltd. Amino acid sequence of AR1:

```
 1   2   3   4   5   6   7   8   9   10
Ala-Asp-Ile-Ser-Gln-Trp-Ala-Gly-Pro-Leu
```

In order to examine acylamino acid digested with acylamino acid-releasing enzyme, fraction Nos. 1 through 20 were hydrolyzed at 110°C for 24 hours with 0.2 ml of constant boiling point hydrochloric acid containing 0.1% thioglycolic acid and their amino acids were analyzed. In fraction Nos. 3 and 4, Ala was detected. In the other fractions, any significant amino acid was not detected. The $NH_2$-terminal amino acid of "HCNP" was thus identified as Ala.

The foregoing results reveal that "HCNP" was a peptide, the $NH_2$-terminal amino acid of which was acylated and which had the following amino acid sequence:

```
 1   2   3   4   5   6   7   8   9   10  11
Ala-Ala-Asp-Ile-Ser-Gln-Trp-Ala-Gly-Pro-Leu
```

In order to examine the kind of acylation, the acylalanine contained in fraction Nos. 3 and 4 was compared with various acylalanine derivatives were analyzed using high performance liquid chromatography of the reverse phase system (Method in Enzymology). As the result of comparing them in retention time, the acylalanine had retention time coincident with that of acetylalanine. It is thus made clear that the $NH_2$-terminal amino acid Ala of "HCNP" was acetylated.

Furthermore, Acetyl-Ala-Ala-Asp-Ile-Ser-Gln-Trp-Ala-Gly-Pro-Leu obtained by the peptide synthesis was analyzed by high performance liquid chromatography on reverse phase TSK-120T column. As the result, the retention time was coincident with that of the synthetic peptide.

## Example 3

### Synthesis of HCNP(8-11):

As an intermediate of chemically synthesizing HCNP and as an authentic sample for structural analysis of "HNCP", the peptide corresponding to the amino acids 8 to 11 at the amino acid sequence of "HCNP" (abbreviated as HCNP(8-11)) was synthesized.

Chloromethylated polystyrene vinylbenzene resin (1% divinylbenzene with an initial chloride loading of 0.68 mmol/g of the resin) of 100 to 200 mesh was e ployed.

Upon synthesis of polypeptide, 3.05 g of Boc-Leu-OH was dissolved in a mixture of 20 ml of ethyl alcohol and 7 ml of water and pH was adjusted to 7 with 20% cesium carbonate solution. The solution was concentrated in vacuo and desicated. To the residue was added 120 ml of DMF and 15 g of the chloromethylated resin. The mixture was stirred at 50°C for 12 hours and then at room temperature for further 12 hours to esterify. The resulting Boc-Leu-O-resin was filtered and washed sequentially with DMF, 90% DMF, DMF and ethyl alcohol and then desicated. Yield, 16.9 g.

Six grams of this Boc-Leu-O-resin were charged in a solid phase synthesis reactor. Following Schedule 1 described hereinafter, Boc-Pro-OH, Boc-Gly-OH and Boc-Ala-OH were successively coupled with the resin. Thus, 6.7 g of HCNP(8-11) peptide resin was obtained.

To 1.1 g of this HCNP(8-11) peptide resin were added 3 ml of anisole, 0.5 ml of ethylmethyl sulfide and 20 ml of anhydrous hydrogen fluoride. The mixture was reacted at -20°C for 60 minutes and then at 0°C

for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in water. The solution was applied to reverse phase YMC-A363(S-5) ODS column (30 $\phi$ x 250 mm) previously equilibrated with 0.1% trifluoroacetic acid. After the column was washed with 0.1% trifluoroacetic acid aqueous solution, the peptide was eluted with linear gradient of 0 to 18% acetonitrile at a flow rate of 7.0 ml/min. The eluent was monitored at A230 nm. The fractions containing the desired product were collected and lyophilized to give 182.3 mg of HCNP(8-11).

The thus obtained HCNP(8-11) was eluted at retention time of 14.7 minutes with linear density gradient of 10-50% aqueous acetonitrile containing 0.1% trifluoroacetic acid through reverse phase YMC-A211-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110°C for 24 hours
Analysis method: PICO-TAG (reverse phase-PTC amino acid) method
Result:
Gly: 1.00 (1)
Ala: 0.90 (1)
Pro: 0.95 (1)
*Leu: 1.00 (1)
*Leu was used as a standard amino acid. The values in parentheses indicate calculated values:

Schedule 1

| | Steps | Time (min.) x Treatment times |
|---|---|---|
| 1. | Washing with methylene chloride, 60 ml | 2 x 3 |
| 2. | Deprotection with 50% TFA, 5% ethanedithiol, 45% methylene chloride (V/V), 60 ml | 3 x 1<br>20 x 1 |
| 3. | Washing with methylene chloride, 60 ml | 2 x 2 |
| 4. | Washing with methanol, 60 ml | 2 x 2 |
| 5. | Neutralization with 10% triethylamine, 90% methylene chloride (V/V), 60 ml | 1 x 1 |
| 6. | Washing with methanol, 60 ml | 2 x 1 |
| 7. | Neutralization with 10% triethylamine, 90% methylene chloride (V/V), 60 ml | 1 x 1 |
| 8. | Washing with methanol, 60 ml | 2 x 2 |
| 9. | Washing with methylene chloride, 60 ml | 2 x 3 |

| | | |
|---|---|---|
| 10. | Coupling by the use of various amino group-protected amino acids (6 mmols), additive (HOBt or HONp) 50% DMF-50% methylene chloride (V/V), 30 ml | 5 x 1 |
| | Solution of DCC (6 mmols) in methylene chloride, 12 ml | 120 x 1 |
| 11. | Washing with 50% DMF, 50% methylene chloride (V/V), 60 ml | 2 x 2 |
| 12. | Washing with methanol, 60 ml | 2 x 1 |
| 13. | Neutralization with 10% triethylamine, 90% methylene chloride (V/V), 60 ml | 1 x 1 |
| 14. | Washing with methanol, 60 ml | 2 x 2 |
| 15. | Washing with methylene chloride, 60 ml | 2 x 2 |
| 16. | Acetylation with 25% acetic anhydride, 75% methylene chloride (V/V), 60 ml | 15 x 1 |
| 17. | Washing with methylene chloride 60 ml | 2 x 2 |
| 18. | Washing with methanol, 60 ml | 2 x 2 |

After the coupling reaction in the step 10, where the resin was subjected to ninhydrin test to show positive blue indicative of incompleteness of the coupling reaction, the coupling reaction was repeated using the same reagents under the same conditions except that DMF or 1-methyl-2-pyrrolidinone was used instead of 50% DMF-50% methylene chloride (V/V) and that the coupling reaction was carried out for a maximum of 12 hours.

Example 4

Synthesis of HCNP(8-11)$NH_2$:

As an authentic sample for structural analysis of "HCNP", HCNP(8-11)$NH_2$ was synthesized.

4-Methylbenzhydrylamine resin (1% divinylbenzene with an initial amino group loading of 0.73 mmol/g of the resin) of 200 to 400 mesh was employed.

Six grams of this resin were charged in a solid phase synthesis reactor. Following Schedule 1 described in Example 3, synthesis started from Step 3 to couple Boc-Leu-OH, Boc-Pro-OH, Boc-Gly-OH and Boc-Ala-OH successively with the resin. Thus, 7.6 g of HCNP(8-11)$NH_2$ peptide resin was obtained.

To 1 g of this HCNP(8-11)$NH_2$ peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was

lyophilized to give a crude peptide, which was dissolved in water. The solution was applied to reverse phase YMC-A363(S-5) ODS column (30 $\phi$ x 250 mm) previously equilibrated with 0.1% trifluoroacetic acid. After the column was washed with 0.1% aqueous trifluoroacetic acid aqueous solution, the peptide was eluted with linear gradient of 0 to 17% acetonitrile at a flow rate of 7.0 ml/min. The eluent was monitored at A230 nm. The fractions containing the desired product were collected and lyophilized to give 177.4 mg of HCNP(8-11)$NH_2$.

The thus obtained HCNP(8-11)$NH_2$ was eluted at retention time of 12.5 minutes with linear density gradient of 10-50% aqueous acetonitrile containing 0.1% trifluoroacetic acid through reverse phase YMC-A211-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Gly: 1.01 (1)
Ala: 0.91 (1)
Pro: 0.97 (1)
*Leu: 1.00 (1)
*Leu was used as a standard amino acid. The values in parentheses indicate calculated values.

Example 5

Synthesis of Ac-HCNP:

The HCNP(8-11) peptide resin, 4.5 g, described in Example 3 was charged in a solid phase synthesis reactor. Following Schedule 1 described in Example 3, Boc-Trp-OH, Boc-Gln-OH, Boc-Ser(Bzl)-OH, Boc-Ile-OH, Boc-Asp(OcHex)-OH and Boc-Ala-OH were successively coupled with the resin. After the coupling reaction of the last amino acid and completion of the acetylation step, approximately 3/4 of the resin was withdrawn. Thus, 5.09 g of HCNP(2-11) peptide resin was obtained. The remaining resin was further coupled with Boc-Ala-OH following Schedule 1. Approximately 1/2 of the resin was withdrawn and 0.72 g f-HCNP peptide resin was obtained. With the remaining resin, the procedures of Steps 1 through 9 and then Steps 16 through 18 of Schedule 1 were repeated to deprotect and effect acetylation. Thus, 0.97 g of Ac-HCNP peptide resin was obtained.

To 0.97 g of this Ac-HCNP peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 0.5% trifluoroacetic acid aqueous solution. The solution was applied to reverse phase YMC-A363(S-5) ODS column (30 $\phi$ x 250 mm) previously equilibrated with 0.1% trifluoroacetic acid aqueous solution. After the column was washed with 0.1% trifluoroacetic acid aqueous solution, the peptide was eluted with linear gradient of 0 to 33% acetonitrile at a flow rate of 7.0 ml/min. The eluent was monitored at A280 nm. The fractions containing the desired product were collected and lyophilized to give 76.6 mg of Ac-HCNP.

The thus obtained Ac-HCNP was eluted at retention time of 28.9 minutes with linear density gradient of 10-50% aqueous acetonitrile containing 0.1% trifluoroacetic acid through reverse phase YMC-A211-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Asp: 0.92 (1)
Glu: 0.93 (1)
Ser: 0.91 (1)
Gly: 1.03 (1)

Ala: 2.67 (3)
Pro: 1.04 (1)
Ile: 1.04 (1)
*Leu: 1.00 (1)
Trp: 0.58 (1)
*Leu was used as a standard amino acid. The values in parentheses indicate calculated values.

Example 6

Synthesis of f-HCNP:

To 0.72 g of the f-HCNP peptide resin described in Example 5 were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 20 ml of 50% acetic acid aqueous solution. After diluting with 80 ml of water, the system was applied to reverse phase YMC-A363(S-5) ODS column (30 $\phi$ x 250 mm) previously equilibrated with 0.1% trifluoroacetic acid aqueous solution. After the column was washed with 0.1% trifluoroacetic acid aqueous solution, the peptide was eluted with linear gradient of 0 to 30% acetonitrile at a flow rate of 7.0 ml/min. The eluent was monitored at A280 nm. The fractions containing the desired product were collected and lyophilized to give 78.0 mg of f-HCNP.

The thus obtained f-HCNP was eluted at retention time of 25.9 minutes with linear density gradient of 10-50% aqueous acetonitrile containing 0.1% trifluoroacetic acid through reverse phase YMC-A211-ODS column (4.6 $\phi$ x 250 mm). The thus obtained peptide was identified by amino acid analysis as follows.
Amino acid analysis
Hydrolysis: 4N Methanesulfonic acid, 2% tryptamine, at 110°C for 24 hours
Analysis method: PICO-TAG (reverse phase-PTC amino acid) method
Result:
Asp: 0.93 (1)
Glu: 0.91 (1)
Ser: 0.91 (1)
Gly: 1.00 (1)
Ala: 2.63 (3)
Pro: 1.02 (1)
Ile: 1.05 (1)
*Leu: 1.00 (1)
Trp: 0.60 (1)
*Leu was used as a standard amino acid. The values in parentheses indicate calculated values.

Example 7

Synthesis of Ac-HCNP(1-11)$NH_2$:

MBHA resin (1% divinylbenzene with an initial amino group loading of 0.73 mmol/g of the resin) of 200 to 400 mesh was employed.

Six grams of this resin were charged in a solid phase synthesis reactor. Following Schedule 1 described in Example 3, synthesis started from Step 3 to couple Boc-Leu-OH, Boc-Pro-OH, Boc-Gly-OH and Boc-Ala-OH successively with the resin. Thus, 7.6 g of HCNP(8-11)$NH_2$ peptide resin as an intermediate was obtained. The half (3.8 g) of the resin was further coupled with Boc-Trp-OH, Boc-Gln-OH, Boc-Ser-(Bzl)-OH, Boc-Ile-OH, Boc-Asp(OcHex)-OH and Boc-Ala-OH successively. After the coupling reaction of the last amino acid and completion of the acetylation step, approximately 3/4 of the resin was withdrawn. The remaining resin was further coupled with Boc-Ala-OH following Schedule 1. Approximately 1/2 of the resin

was withdrawn. With the remaining resin, the procedures of Steps 1 through 9 and then Steps 16 through 18 of Schedule 1 were repeated to deprotect and effect acetylation. Thus, 0.81 g of Ac-HCNP(1-11)$NH_2$ peptide resin was obtained.

To 0.81 g of this Ac-HCNP(1-11)$NH_2$ peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 50 ml of 0.1% TFA aqueous solution. The solution was applied to reverse phase YMC-A363(S-5) ODS COLUMN (30 $\phi$ x 250 mm) previously equilibrated with 0.1% TFA aqueous solution. After the column was washed with 0.1% TFA aqueous solution, the peptide was eluted with linear gradient of 0 to 33% acetonitrile for 180 minutes at a flow rate of 7.0 ml/min. The eluent was monitored at A280 nm. The fractions containing the desired product were collected and lyophilized to give 169.6 mg of Ac-HCNP(1-11)$NH_2$.

The thus obtained Ac-HCNP(1-11)$NH_2$ was eluted at retention time of 28.6 minutes with linear density gradient of 10-50% aqueous acetonitrile containing 0.1% TFA through reverse phase YMC-AM-303(s-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Asx: 0.91 (1)
Glx: 0.90 (1)
Ser: 0.89 (1)
Gly: 1.02 (1)
Ala: 2.56 (3)
Pro: 1.01 (1)
Ile: 1.02 (1)
*Leu: 1.00 (1)
Trp: 0.57 (1)
*Leu was used as a standard amino acid. The values in parentheses indicate calculated values.

## Example 8

Synthesis of Ac-HCNP(1-10)$NH_2$:

MBHA resin (1% divinylbenzene with an initial amino group loading of 0.42 mmol/g of the resin) of 100 to 200 mesh was employed.

Six grams of this resin were charged in a solid phase synthesis reactor. Following Schedule 1 described in Example 3, synthesis started from Step 3 to couple Boc-Pro-OH, Boc-Gly-OH, Boc-Ala-OH, Boc-Trp-OH, Boc-Gln-OH, Boc-Ser(Bzl)-OH, Boc-Ile-OH, Boc-Asp(OcHex)-OH and Boc-Ala-OH with the resin successively using an equimolar amount of DCC. After introducing Boc-Ser(Bzl)-OH, Boc-Ile-OH and Boc-Asp(OcHex)-OH, respectively, in the course of coupling reaction, a part of the resin was withdrawn. Furthermore, after the coupling reaction of the last amino acid and completion of the acetylation step, a part of the resin was withdrawn. Thus, 4.16 g of HCNP(2-10)$NH_2$ peptide resin was obtained. The remaining resin was further coupled with Boc-Ala-OH following Schedule 1. Approximately 1/2 of the resin was withdrawn. With the remaining resin, the procedures of Steps 1 through 9 and then steps 16 through 18 of Schedule 1 were repeated to deprotect and effect acetylation. Thus, 1.26 g of Ac-HCNP(1-10)$NH_2$ peptide resin was obtained.

To 1.26 g of this Ac-HCNP(1-10)$NH_2$ peptide resin were added 3 ml of anisole, 0.5 ml of ethylmethyl sulfide and 20 ml of anhydrous hydrogen fluride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 10% acetic acid aqueous solution. After stirring for 30 minutes, the resin was filtered off and washed with 100 ml of 10% acetic acid aqueous solution. The filtrate was

lyophilized to give a crude peptide, which was dissolved in 100 ml of 10% acetic acid aqueous solution. The solution was applied to reverse phase YMC-A363(S-5) ODS column (30 $\phi$ x 250 mm) previously equilibrated with 0.1% TFA aqueous solution. After the column was washed with 0.1% TFA aqueous solution, the peptide was eluted with linear gradient of 0 to 25% acetonitrile for 180 minutes at a flow rate of 7.0 ml/min. The eluent was monitored at A280 nm. The fractions containing the desired product were collected and lyophilized to give 139.5 mg of Ac-HCNP(1-10)$NH_2$.

The thus obtained Ac-HCNP(1-10)$NH_2$ was eluted at retention time of 22.6 minutes with linear density gradient of 10-50% aqueous acetonitrile containing 0.1% TFA through reverse phase YMC-A363(S-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110 °C for 24 hours
Analysis method: PICO-TAG (reverse phase-PTC amino acid) method
Result:
Asx: 0.99 (1)
Glx: 0.92 (1)
Ser: 0.95 (1)
Gly: 0.96 (1)
*Ala: 3.00 (3)
Pro: 0.99 (1)
Ile: 1.00 (1)
Trp: 0.91 (1)
*Ala was used as a standard amino acid. The values in parentheses indicate calculated values.

Example 9

Synthesis of Ac-HCNP(1-9)$NH_2$:

MBHA resin (1% divinylbenzene with an initial amino group loading of 0.64 mmol/g of the resin) of 100 to 200 mesh was employed.

Six grams of this resin were charged in a solid phase synthesis reactor. Following Schedule 1 described in Example 3, synthesis started from Step 3 to couple Boc-Gly-OH, Boc-Ala-OH, Boc-Trp-OH, Boc-Gln-OH, Boc-Ser(Bzl)-OH, Boc-Ile-OH and Boc-Asp(OcHex)-OH with the resin successively. After introducing Boc-Gln-OH, Boc-Ser(Bzl)-OH and Boc-Ile-OH, respectively, during the course of coupling reaction, a part of the resin was withdrawn. Furthermore, after the coupling reaction of the last amino acid and completion of the acetylation step, a part of the resin was withdrawn. Thus, 1.42 g of HCNP(3-9)$NH_2$ peptide resin was obtained. The remaining resin was further coupled with Boc-Ala-OH following Schedule 1. A part of the resin and the remaining resin was further coupled with Boc-Ala-OH following Schedule 1. Approximately the half of the resin was withdrawn. With the remaining resin, the procedures of Steps 1 through 9 and then Steps 16 through 18 of Schedule 1 were repeated to deprotect and effect acetylation. Thus, 1.06 g of Ac-HCNP(1-9) $NH_2$ peptide resin was obtained.

To 1.06 g of this Ac-HCNP(1-9)$NH_2$ peptide resin were added 3 ml of anisole, 0.5 ml of ethylmethyl sulfide and 20 ml of anhydrous hydrogen fluoride. The mixture was reacted at -20 °C for 60 minutes and then at 0 °C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 10% acetic acid aqueous solution. After stirring for 30 minutes, the resin was filtered off and washed with 100 ml of 10% acetic acid aqueous solution. The filtrate was lyophilized to give a crude peptide, which was dissolved in 150 ml of 10% acetic acid aqueous solution. The solution was applied to reverse phase YMC-A363(S-5)-ODS column (30 $\phi$ x 250 mm) previously equilibrated with 0.1% TFA aqueous solution. After the column was washed with 0.1% TFA aqueous solution, the peptide was eluted with linear gradient of 0 to 26% acetonitrile for 180 minutes at a flow rate of 7.0 ml/min. The eluent was monitored at A280 nm. The fractions containing the desired product were collected and lyophilized to give 37.0 mg of Ac-HCNP(1-9)$NH_2$.

The thus obtained Ac-HCNP(1-9)$NH_2$ was eluted at retention time of 23.0 minutes with linear density gradient of 10-50% aqueous acetonitrile containing 0.1% TFA through reverse phase YMC-AM3-3(S-5)-ODS column (4.6 $\phi$ x 250 mm). The thus obtained peptide was identified by amino acid analysis as follows.

Amino acid analysis

Hydrolysis: 4N Methanesulfonic acid, 2% tryptamine, at 110° C for 24 hours
Analysis method: PICO-TAG (reverse phase-PTC amino acid) method
Result:
Asx: 1.05 (1)
Glx: 1.04 (1)
Ser: 0.98 (1)
Gly: 1.03 (1)
*Ala: 3.00 (3)
Ile: 1.02 (1)
Trp: 0.54 (1)
*Ala was used as a standard amino acid. The values in parentheses indicate calculated values.

Example 10

Synthesis of Ac-HCNP(1-8)$NH_2$:

MBHA resin (1% divinylbenzene with an initial amino group loading of 0.64 mmol/g of the resin) of 100 to 200 mesh was employed.

Six grams of this resin were charged in a solid phase synthesis reactor. Following Schedule 1 described in Example 3, synthesis started from Step 3 to couple Boc-Ala-OH, Boc-Trp-OH, Boc-Gln-OH, Boc-Ser(Bzl)-OH and Boc-Ile-OH with the resin successively. After introducing Boc-Ser(Bzl)-OH during the course of coupling reaction, a part of the resin was withdrawn. Furthermore, after the coupling reaction of the last amino acid and completion of the acetylation step, a part of the resin was withdrawn. Thus, 1.16 g of HCNP(4-8)$NH_2$ peptide resin was obtained. The remaining resin was further coupled with Boc-Asp-(OcHex)-OH following Schedule 1. A part of the resin was withdrawn and the remaining resin was further coupled with Boc-Ala-OH following Schedule 2 later described. A part of the resin was withdrawn and 0.76 g of HCNP(2-8)$NH_2$ peptide resin was obtained. The remaining resin was further coupled with Boc-Ala-OH following Schedule 2. Approximately the half of the resin was withdrawn. With the remaining resin, the procedures of Steps 1 through 9 and then Steps 16 through 18 of Schedule 2 were repeated to deprotect and effect acetylation. Thus, 1.56 g of Ac-HCNP(1-8)$NH_2$ peptide resin was obtained.

To 1.56 g of this Ac-HCNP(1-8) $NH_2$ peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes the resin was filtered off and washed with 100 ml of 5% acetic acid aqueous solution. The filtrate was lyophilized to give a crude peptide, which was dissolved in 400 ml of 10% acetic acid aqueous solution. The solution was applied to reverse phase YMC-SH-343-5(S-5)-ODS column (20 $\phi$ x 250 mm) previously equilibrated with 0.1% TFA aqueous solution. After the column was washed with 0.1% TFA aqueous solution, the peptide was eluted with linear gradient of 0 to 25% acetonitrile for 180 minutes and further to 31% acetonitrile for 60 minutes at a flow rate of 7.0 ml/min. The eluent was monitored at A280 nm. The fractions containing the desired product were collected and lyophilized to give 39.1 mg of Ac-HCNP(1-8)-$NH_2$.

The thus obtained Ac-HCNP(1-8)$NH_2$ was eluted at retention time of 23.5 minutes with linear density gradient of 10-50% aqueous acetonitrile containing 0.1% TFA through reverse phase YMC-AM303(S-5)-ODS column (4.6 $\phi$ x 250 mm). The thus obtained peptide was identified by amino acid analysis as follows.
Amino acid analysis
Hydrolysis: 4N Methanesulfonic acid, 2% tryptamine, at 110° C for 24 hours
Analysis method: PICO-TAG (reverse phase-PTC amino acid) method
Result:
Asx: 1.01 (1)
Glx: 0.99 (1)
Ser: 0.95 (1)
*Ala: 3.00 (3)
Ile: 1.00 (1)
Trp: 0.68 (1)

*Ala was used as a standard amino acid. The values in parentheses indicate calculated values.

Schedule 2

| Steps | Time (min.) x Treatment times |
|---|---|
| 1. Washing with methylene chloride, 30 ml | 2 x 3 |
| 2. Deprotection with 50% TFA, 5% ethanedithiol, 45% methylene chloride (V/V), 30 ml | 3 x 1<br>20 x 1 |
| 3. Washing with methylene chloride, 30 ml | 2 x 2 |
| 4. Washing with methanol, 30 ml | 2 x 2 |
| 5. Neutralization with 10% triethylamine, 90% methylene chloride (V/V), 30 ml | 1 x 1 |
| 6. Washing with methanol, 30 ml | 2 x 1 |
| 7. Neutralization with 10% triethylamine, 90% methylene chloride (V/V), 30 ml | 1 x 1 |
| 8. Washing with methanol, 30 ml | 2 x 2 |
| 9. Washing with methylene chloride, 30 ml | 2 x 3 |
| 10. Coupling by the use of various amino group-protected amino acids (3 mmols), additive (HOBt or HONp) 50% DMF, 50% methylene chloride (V/V), 15 ml | 5 x 1 |
| Solution of DCC (3 mmols) in methylene chloride, 6 ml | 120 x 1 |

| | | |
|---|---|---|
| 11. | Washing with 50% DMF, 50% methylene chloride (V/V), 30 ml | 2 x 2 |
| 12. | Washing with methanol, 30 ml | 2 x 1 |
| 13. | Neutralization with 10% triethyl-amine, 90% methylene chloride (V/V), 30 ml | 1 x 1 |
| 14. | Washing with methanol, 30 ml | 2 x 2 |
| 15. | Washing with methylene chloride, 30 ml | 2 x 2 |
| 16. | Acetylation with 25% acetic anhydride, 75% methylene chloride (V/V), 30 ml | 15 x 1 |
| 17. | Washing with methylene chloride 30 ml | 2 x 2 |
| 18. | Washing with methanol, 30 ml | 2 x 2 |

After the coupling reaction in the step 10, where the resin was subjected to ninhydrin test to show positive blue indicative of incompleteness of the coupling reaction, the coupling reaction was repeated using the same reagents under the same conditions except that DMF or 1-methyl-2-pyrrolidinone was used instead of 50% DMF-50% methylene chloride (V/V) and that the coupling reaction was carried out for a maximum of 12 hours.

Example 11

Synthesis of Ac-HCNP(1-7):

Chloromethylated polystyrene vinylbenzene resin (1% divinylbenzene with an initial chloride loading of 0.68 mmol/g of the resin) of 100 to 200 mesh was employed.

Upon synthesis of Ac-HCNP(1-7), 2.48 g of Boc-Trp-OH was firstly dissolved in a mixture of 15 ml of ethyl alcohol and 5 ml of water and, pH was adjusted to 7 with 20% cesium carbonate solution. The solution was concentrated in vacuo and desicated. To the residue were added 80 ml of DMF and 10 g of the chloromethylated resin. The mixture was stirred at 50° C for 10 hours and then at room temperature for further 12 hours to esterify. The resulting Boc-Trp-O-resin was filtered and washed sequentially with DMF, 90% DMF, DMF and ethyl alcohol and then desicated. Yield, 11.1 g.

Six grams of this Boc-Trp-O-resin were charged in a solid phase synthesis reactor. Following Schedule 1 described in Example 3, Boc-Gln-OH, Boc-Ser(Bzl)-OH, Boc-Ile-OH, Boc-Asp(OcHex)-OH and Boc-Ala-OH were successively coupled with the resin. After introducing Boc-Ile-OH and Boc-Asp(OcHex)-OH during the course of coupling reaction, a part of the resin was withdrawn. Furthermore, after coupling reaction of the last Boc-Ala-OH and completion of the acetylation step, approximately 1/2 of the resin was withdrawn to give 2.83 g of HCNP(2-7) peptide resin. The remaining resin was further coupled with Boc-Ala-OH following Schedule 2 described in Example 10. A part of the resin was withdrawn and 2.08 g of HCNP(1-7) peptide resin was obtained. With the remaining resin, the procedures of Steps 1 through 9 and then Steps 16 through 18 of Schedule 2 were repeated to deprotect and effect acetylation. Thus, 0.90 g of Ac-HCNP(1-7) peptide resin was obtained.

To 0.90 g of this HCNP(1-7) peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to

the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 200 ml of 10% acetic acid aqueous solution. The solution was applied to reverse phase YMC-A363(S-5) ODS column (30 φ x 250 mm) previously equilibrated with 0.1% TFA aqueous solution. After the column was washed with 0.1% TFA aqueous solution, the peptide was eluted with linear gradient of 0 to 28% acetonitrile for 60 minutes at a flow rate of 7.0 ml/min. The eluent was monitored at A280 nm. The fractions containing the desired product were collected and lyophilized to give 54.1 mg of Ac-HCNP(1-7).

The thus obtained Ac-HCNP(1-7) was eluted at retention time of 24.4 minutes with linear density gradient of 10-50% aqueous acetonitrile containing 0.1% TFA through reverse phase YMC-AM303(S-5)-ODS column (30 φ x 250 mm). The thus obtained peptide was identified by amino acid analysis as follows.

Amino acid analysis
Hydrolysis: 4N Methanesulfonic acid, 2% tryptamine, at 110 °C for 24 hours
Analysis method: PICO-TAG (reverse phase-PTC amino acid) method
Result:
Asx: 1.05 (1)
Glx: 1.03 (1)
Ser: 1.01 (1)
*Ala: 2.00 (2)
Ile: 1.10 (1)
Trp: 0.63 (1)
*Ala was used as a standard amino acid. The values in parentheses indicate calculated values:

Example 12

Synthesis of HCNP(1-7):

To 1.00 g of HCNP(1-7) peptide resin described in Example 11 were added 3 ml of anisole, 0.5 ml of ethylmethyl sulfide and 20 ml of anhydrous hydrogen fluoride. The mixture was reacted at -20 °C for 60 minutes and then at 0 °C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 50 ml of 10% acetic acid aqueous solution. The solution was applied to reverse phase YMC-A363(S-5)-ODS column (30 φ x 250 mm) previously equilibrated with 0.1% TFA aqueous solution. After the column was washed with 0.1% TFA aqueous solution, the peptide was eluted with linear gradient of 0 to 25% acetonitrile for 120 minutes at a flow rate of 7.0 ml/min. The eluent was monitored at A280 nm. The fractions containing the desired product were collected and lyophilized to give 67.1 mg of HCNP(1-7).

The thus obtained HCNP(1-7) was eluted at retention time of 21.4 minutes with linear density gradient of 10-50% aqueous acetonitrile containing 0.1% TFA through reverse phase YMC-AM303(S-5)-ODS column (4.6 φ x 250 mm). The thus obtained peptide was identified by amino acid analysis as follows.

Amino acid analysis
Hydrolysis: 4N Methanesulfonic acid, 2% tryptamine, at 110 °C for 24 hours
Analysis method: PICO-TAG (reverse phase-PTC amino acid) method
Result:
Asx: 1.02 (1)
Glx: 1.00 (1)
Ser: 0.97 (1)
*Ala: 2.00 (2)
Ile: 1.11 (1)
Trp: 0.70 (1)
*Ala was used as a standard amino acid. The values in parentheses indicate calculated values:

Example 13

Synthesis of For-HCNP(1-7):

Upon synthesis of For-HCNP(1-7), 4.46 g of alanine was firstly dissolved in 105 ml of 98% formic acid and, 35 ml of acetic anhydride was then dropwise added to the solution under ice cooling in the period of 10 min. After stirring for further an hour, 80 ml of water was added to the reaction mixture and concentrated in vacuo. Diethyl ether was added to the reside to crystallize. The crystals were taken by filtration, washed with diethyl ether and desicated to give 4.89 g of For-Ala-OH.

The HCNP(2-7) peptide resin, 1.00 g, described in Example 11 was charged in a solid phase synthesis reactor and For-Ala-OH described above was coupled therewith following Schedule 2 described in Example 10. Thus, 1.03 g of For-HCNP(1-7) peptide resin was obtained.

To 1.03 g of this For-HCNP(1-7) peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 100 ml of 10% acetic acid aqueous solution. The solution was applied to reverse phase YMC-A363(S-5)-ODS column (30 $\phi$ x 250 mm) previously equilibrated with 0.1% TFA aqueous solution. After the column was washed with 0.1% TFA aqueous solution, the peptide was eluted with linear gradient of 0 to 28% acetonitrile for 120 minutes at a flow rate of 7.0 ml/min. The eluent was monitored at A280 nm. The fractions containing the desired product were collected and lyophilized to give 33.0 mg of For-HCNP(1-7).

The thus obtained For-HCNP(1-7) was eluted at retention time of 24.0 minutes with linear density gradient of 10-50% aqueous acetonitrile containing 0.1% TFA through reverse phase YMC-AM303(S-5)-ODS column (4.6 $\phi$ x 250 mm). The thus obtained peptide was identified by amino acid analysis as follows.
Amino acid analysis
Hydrolysis: 4N Methanesulfonic acid, 2% tryptamine, at 110° C for 24 hours
Analysis method: PICO-TAG (reverse phase-PTC amino acid) method
Result:
Asx: 1.02 (1)
Glx: 1.01 (1)
Ser: 0.99 (1)
*Ala: 2.00 (2)
Ile: 1.11 (1)
Trp: 0.54 (1)
*Ala was used as a standard amino acid. The values in parentheses indicate calculated values:

Example 14

Synthesis of HCNP(1-7)$NH_2$:

MBHA resin (1% divinylbenzene with an initial amino group loading of 0.73 mmol/g of the resin) of 200 to 400 mesh was employed.

Six grams of this resin were charged in a solid phase synthesis reactor. Following Schedule 1 described in Example 3, synthesis started from Step 3 to couple Boc-Trp-OH, Boc-Gln-OH, Boc-Ser(Bzl)-OH, Boc-Ile-OH, Boc-Asp(OcHex)-OH, Boc-Ala-OH and Boc-Ala-OH with the resin in this order. After introducing Boc-Ile-OH, Boc-Asp(OcHex)-OH and Boc-Ala-OH during the course of coupling reaction, a part of the resin was withdrawn. Thus, 3.34 g of HCNP(1-7)$NH_2$ peptide resin was obtained.

To 0.83 g of this HCNP(1-7)$NH_2$ peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 m of diethyl ether. To the residue was added 100 ml of 5% acetic acid aqueous solution. After stirring for 30 minutes, the resin

was filtered off and washed with 100 ml of 5% acetic acid aqueous solution. The filtrate was lyophilized to give a crude peptide, which was dissolved in 200 ml of 2.5% acetic acid aqueous solution. The solution was applied to reverse phase YMC-ODS(S-10/20) column (30 $\phi$ x 300 mm) previously equilibrated with 0.1% TFA aqueous solution. After the column was washed with 0.1% TFA aqueous solution, the peptide was eluted with linear gradient of 0 to 18% acetonitrile for 200 minutes at a flow rate of 6.0 ml/min. The eluent was monitored at A280 nm. The fractions containing the desired product were collected and lyophilized to give 71.9 mg of HCNP(1-7)$NH_2$ as primary purification product. This primary purification product of HCNP-(1-7)$NH_2$ was further dissolved in 50 ml of 2% acetic acid aqueous solution and applied to reverse phase YMC-SH-343-5(S-5) ODS column (20 $\phi$ x 500 mm) previously equilibrated with 0.1% TFA aqueous solution. After the column was washed with 0.1% TFA aqueous solution, the peptide was eluted with linear gradient of 0 to 16% acetonitrile for 180 minutes, then to 19% acetonitrile for 60 minutes and further to 21% acetonitrile for 60 minutes at a flow rate of 7.0 ml/min. The eluent was monitored at A280 nm. The fractions containing the desired product were collected and lyophilized to give 26.8 mg of HCNP(1-7)$NH_2$.

The thus obtained HCNP(1-7)$NH_2$ was eluted at retention time of 19.0 minutes with linear density gradient of 10-50% aqueous acetonitrile containing 0.1% TFA through reverse phase YMC-AM303(S-5)-ODS column (4.6 $\phi$ 250 mm). The thus obtained peptide was identified by amino acid analysis as follows.

Amino acid analysis
Hydrolysis: 4N Methanesulfonic acid, 2% tryptamine, at 110°C for 24 hours
Analysis method: PICO-TAG (reverse phase-PTC amino acid) method
Result:
Asx: 1.05 (1)
Gly: 1.01 (1)
Ser: 1.00 (1)
*Ala: 2.00 (2)
Ile: 1.08 (1)
Trp: 0.78 (1)
*Ala was used as a standard amino acid. The values in parentheses indicate calculated values:

## Example 15

Synthesis of HCNP(2-11):

To 1.00 g of HCNP(2-11) peptide resin described in Example 5 were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 300 ml of 10% acetic acid aqueous solution. The solution was applied to reverse phase YMC-A363(S-5) ODS column (30 $\phi$ x 250 mm) previously equilibrated with 0.1% TFA aqueous solution. After the column was washed with 0.1% TFA aqueous solution, the peptide was eluted with linear gradient of 0 to 30% acetonitrile for 180 minutes at a flow rate of 7.0 ml/min. The eluent was monitored at A280 nm. The fractions containing the desired product were collected and lyophilized to give 134.2 mg of HCNP(2-11).

The thus obtained HCNP(2-11) was eluted at retention time of 25.9 minutes with linear density gradient of 10-50 aqueous acetonitrile containing 0.1% TFA through reverse phase YMC-AM303(S-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110°C for 24 hours
Analysis method: PICO-TAG (reverse phase-PTC amino acid) method
Result: Asx:
0.92 (1)
Glx: 0.91 (1)
Ser: 0.89 (1)
Gly: 0.99 (1)
Ala: 1.76 (2)

Pro: 1.00 (1)
Ile: 1.02 (1)
*Leu: 1.00 (1)
Trp: 0.64 (1)
*Leu was used as a standard amino acid. The values in parentheses indicate calculated values.

Example 16

Synthesis of HCNP(4-11)

Chloromethylated polystyrene vinylbenzene resin (1% divinylbenzene with an initial chloride loading of 0.68 mmol/g of the resin) of 100 to 200 mesh was employed.

Upon synthesis of HCNP(4-11), 4.07 g of Boc-Leu-OH was dissolved in a mixture of 30 ml of ethyl alcohol and 10 ml of water and, pH was adjusted to 7 with 20% cesium carbonate aqueous solution. The solution was concentrated in vacuo and desicated. To residue was added 160 ml of DMF and 20 g of the chloromethylated resin. The mixture was stirred at 50° C for 12 hours and then at room temperature for further 12 hours to esterify. The resulting Boc-Leu-O-resin was filtered and washed successively with DMF, 90% DMF, DMF and ethyl alcohol and then desicated. Yield, 23.4 g.

Twenty grams of this Boc-Leu-O-resin were charged in a solid phase synthesis reactor. Following Schedule 3 described hereinafter, Boc-Pro-OH, Boc-Gly-OH, Boc-Ala-OH and Boc-Trp-OH were successively coupled with the resin. A part of the resin was withdrawn and, 1.05 g of HCNP(7-11) peptide resin was obtained. The remaining resin was further coupled with Boc-Gln-OH, following Schedule 3. A part of the resin was withdrawn and, 1.17 g of HCNP(6-11) peptide resin was obtained. The remaining resin was further coupled with Boc-Ser(Bzl)-OH, following Schedule 3. Thus, 24.50 g of HCNP(5-11) peptide resin was obtained. Following Schedule 1 described in Example 3, 6.00 g of this HCNP(5-11) peptide resin was further coupled with Boc-Ile-OH. A part of the resin was withdrawn and, 1.50 g of HCNP(4-11) peptide resin was obtained.

To 0.75 g of this HNCP(4-11) peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 50 ml of 10% acetic acid aqueous solution. The solution was applied to reverse phase YMC-A363(S-5)-ODS column (30 $\phi$ x 250 mm) previously equilibrated with 0.1% TFA aqueous solution. After the column was washed with 0.1% TFA aqueous solution, the peptide was eluted with linear gradient of 0 to 29% acetonitrile for 180 minutes at a flow rate of 7.0 ml/min. the eluent was monitored at A280 nm. The fractions containing the desired product were collected and lyophilized to give 76.1 mg of HCNP(4-11).

The thus obtained DNS-HCNP(4-8)OMe was eluted at retention time of 45.1 minutes with linear density gradient of 10-80% aqueous acetonitrile containing 0.1% TFA through reverse phase YMC-AM303(S-5)-ODS column (4.6 $\phi$ x 250 mm). The thus obtained peptide was identified by amino acid analysis as follows.
Amino acid analysis
Hydrolysis: 4N Methanesulfonic acid, 2% tryptamine, at 110° C for 24 hours
Analysis method: PICO-TAG (reverse phase-PTC amino acid) method
Result:
Glx: 0.98 (1)
Ser: 0.88 (1)
*Ala: 1.00 (1)
Trp: 0.56 (1)
*Ala was used as a standard amino acid. The values in parentheses indicate calculated values. Existence of Ile was confirmed by detection of DNS-Ile.

Schedule 3

| Steps | Time (min.) x Treatment times |
|---|---|
| 1. Washing with methylene chloride, 200 ml | 2 x 3 |
| 2. Deprotection with 50% TFA, 5% ethanedithiol, 45% methylene chloride (V/V), 200 ml | 3 x 1<br>20 x 1 |
| 3. Washing with methylene chloride, 200 ml | 2 x 2 |
| 4. Washing with methanol, 200 ml | 2 x 2 |
| 5. Neutralization with 10% triethylamine, 90% methylene chloride (V/V), 200 ml | 1 x 1 |
| 6. Washing with methanol, 200 ml | 2 x 1 |
| 7. Neutralization with 10% triethylamine, 90% methylene chloride (V/V), 200 ml | 1 x 1 |
| 8. Washing with methanol, 200 ml | 2 x 2 |
| 9. Washing with methylene chloride, 200 ml | 2 x 3 |

| | | |
|---|---|---|
| 10. | Coupling by the use of various amino group-protected amino acids (20 mmols), additive (HOBt) 50% DMF, 50% methylene chloride (V/V), 100 ml | 5 x 1 |
| | Solution of DCC (20 mmols) in methylene chloride, 40 ml | 120 x 1 |
| 11. | Washing with 50% DMF, 50% methylene chloride (V/V), 200 ml | 2 x 2 |
| 12. | Washing with methanol, 200 ml | 2 x 1 |
| 13. | Neutralization with 10% triethylamine, 90% methylene chloride (V/V), 200 ml | 1 x 1 |
| 14. | Washing with methanol, 200 ml | 2 x 2 |
| 15. | Washing with methylene chloride, 200 ml | 2 x 2 |
| 16. | Acetylation with 25% acetic anhydride, 75% methylene chloride (V/V), 200 ml | 15 x 1 |
| 17. | Washing with methylene chloride 200 ml | 2 x 2 |
| 18. | Washing with methanol, 200 ml | 2 x 2 |

After the coupling reaction in the step 10, where the resin was subjected to ninhydrin test to show positive blue indicative of incompleteness of the coupling reaction, the coupling reaction was repeated using the same reagents under the same conditions.

Example 17

Synthesis of HCNP(6-11):

To 0.70 g of HCNP(6-11) peptide resin described in Example 16 were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 30 ml of water. The solution was applied to reverse phase YMC-A363(S-5)-ODS column (30 $\phi$ x 250 mm) previously equilibrated with 0.1% aqueous TFA. After the column was washed with 0.1% TFA aqueous solution, the peptide was eluted with linear gradient of 0 to 27% acetonitrile for 180 minutes at a flow rate of 7.0 ml/min. The eluent was monitored at A280 nm. The fractions containing the desired product were collected and lyophilized to give 63.2 mg of HCNP(6-11) as the primarily purified product. Further 40.0 mg of the primarily purified HCNP(6-11) was dissolved in 0.5 ml of water. The solution was applied to reverse phase YMC-A363(S-5)-ODS column (30 $\phi$ x 250 mm) previously equilibrated with 0.1% aqueous TFA. After the column was washed with

0.1% TFA aqueous solution, the peptide was eluted with linear gradient of 0 to 27% acetonitrile for 60 minutes at a flow rate of 7.0 ml/min. The eluent was monitored at A280 nm. The fractions containing the desired product were collected and lyophilized to give 25.7 mg of HCNP(6-11).

The thus obtained HCNP(6-11) was eluted at retention time of 23.7 minutes with linear density gradient of 10-50% aqueous acetonitrile containing 0.1% TFA through reverse phase YMC-AM303(S-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Glx: 0.96 (1)
Gly: 1.03 (1)
Ala: 0.94 (1)
Pro: 0.93 (1)
*Leu: 1.00 (1)
Trp: 0.63 (1)
*Leu was used as a standard amino acid. The values in parentheses indicate calculated values.

Example 18

Synthesis of HCNP(7-11):

To 0.50 g of HCNP(7-11) peptide resin described in Example 16 were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 50 ml of 10% acetic acid aqueous solution. The solution was applied to reverse phase YMC-A363(S-5)-ODS column (30 $\phi$ x 250 mm) previously equilibrated with 0.1% TFA aqueous solution. After the column was washed with 0.1% TFA aqueous solution, the peptide was eluted with linear gradient of 0 to 28% acetonitrile for 180 minutes at a flow rate of 7.0 ml/min. The eluent was monitored at A280 nm. The fractions containing the desired product were collected and lyophilized to give 44.8 mg of HCNP(7-11).

The thus obtained HCNP(7-11) was eluted at retention time of 24.3 minutes with linear density gradient of 10-50% aqueous acetonitrile containing 0.1% TFA through reverse phase YMC-AM303(S-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Gly: 1.05 (1)
Ala: 0.95 (1)
Pro: 0.96 (1)
*Leu: 1.00 (1)
Trp: 0.62 (1)
*Leu was used as a standard amino acid. The values in parentheses indicate calculated values.

Example 19

Synthesis of HCNP(2-10)$NH_2$:

To 1.00 g of HCNP(2-10)$NH_2$ peptide resin described in Example 8 were added 3 ml of anisole, 0.5 ml of ethylmethyl sulfide and 20 ml of anhydrous hydrogen fluoride. The mixture was reacted at -20° C for 60

minutes and then at 0 °C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 300 ml of 10% acetic acid aqueous solution. The solution was applied to reverse phase YMC-A363(S-5)-ODS column 930 $\phi$ x 250 mm) previously equilibrated with 0.1% TFA aqueous solution. After the column was washed with 0.1% TFA aqueous solution, the peptide was eluted with linear gradient of 0 to 21% acetonitrile for 360 minutes at a flow rate of 7.0 ml/min. The eluent was monitored at A280 nm. The fractions containing the desired product were collected and lyophilized to give 108.0 mg of HCNP(2-10)$NH_2$.

The thus obtained HCNP(2-10) $NH_2$ was eluted at retention time of 19.7 minutes with linear density gradient of 10-50% aqueous acetonitrile containing 0.1% TFA through reverse phase YMC-AM303(S-5)-ODS column (4.6 $\phi$ x 250 mm). The thus obtained peptide was identified by amino acid analysis as follows.
Amino acid analysis
Hydrolysis: 4N Methanesulfonic acid, 2% tryptamine, at 110 °C for 24 hours
Analysis method: PICO-TAG (reverse phase-PTC amino acid) method
Result:
Asx: 0.95 (1)
Glx: 0.92 (1)
Ser: 0.94 (1)
Gly: 0.95 (1)
*Ala: 2.00 (2)
Pro: 0.99 (1)
Ile: 1.00 (1)
Trp: 0.82 (1)
*Ala was used as a standard amino acid. The values in parentheses indicate calculated values.

Example 20

Synthesis of HCNP(2-8)$NH_2$:

To 0.76 g of HCNP(2-8)$NH_2$ peptide resin described in Example 10 were added 3 ml of anisole, 0.5 ml of ethylmethyl sulfide and 20 ml of anhydrous hydrogen fluoride. The mixture was reacted at -20 °C for 60 minutes and then at 0 °C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 100 ml of 10% acetic acid aqueous solution. The solution was applied to reverse phase YMC-SH-343-5(S-5)-ODS column (20 $\phi$ x 250 mm) previously equilibrated with 0.1% TFA aqueous solution. After the column was washed with 0.1% TFA aqueous solution, the peptide was eluted with linear gradient of 0 to 22% acetonitrile for 180 minutes and then to 28% for 60 minutes at a flow rate of 7.0 ml/min. The eluent was monitored at A280 nm. The fractions containing the desired product were collected and lyophilized to give 69.4 mg of HCNP(2-8)$NH_2$.

The thus obtained HCNP(2-8)$NH_2$ was eluted at retention time of 20.4 minutes with linear density gradient of 10-50% aqueous acetonitrile containing 0.1% TFA through reverse phase YMC-AM303(S05)-ODS column (4.6 $\phi$ x 250 mm). The thus obtained peptide was identified by amino acid analysis as follows.
Amino acid analysis
Hydrolysis: 4N Methanesulfonic acid, 2% tryptamine, at 110 °C for 24 hours
Analysis method: PICO-TAG (reverse phase-PTC amino acid) method
Result:
Asx: 0.98 (1)
Glx: 0.97 (1)
Ser: 0.92 (1)
*Ala: 2.00 (2)
Ile: 0.98 (1)
Trp: 0.85 (1)

*Ala was used as a standard amino acid. The values in parentheses indicate calculated values.

Example 21

Synthesis of HCNP(2-7):

To 0.92 g of HCNP(2-7) peptide resin described in Example 11 were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 100 ml of 10% acetic acid aqueous solution. The solution was applied to reverse phase YMC-SH-343-5(S-5) ODS column (20 $\phi$ x 250 mm) previously equilibrated with 0.1% TFA aqueous solution. After the column was washed with 0.1% TFA aqueous solution, the peptide was eluted with linear gradient of 0 to 24% acetonitrile for 180 minutes and further up to 30% for 60 minutes at a flow rate of 7.0 ml/min. The eluent was monitored at A280 nm. The fractions containing the desired product were collected and lyophilized to give 35.8 mg of HCNP(2-7).

The thus obtained HCNP(2-7) was eluted at retention time of 21.9 minutes with linear density gradient of 10-50% aqueous acetonitrile containing 0.1% TFA through reverse phase YMC-AM303(S-5)-ODS column (4.0 $\phi$ x 250 mm). The thus obtained peptide was identified by amino acid analysis as follows.
Amino acid analysis
Hydrolysis: 4N Methanesulfonic acid, 2% tryptamine, at 110° C for 24 hours
Analysis method: PICO-TAG (reverse phase-PTC amino acid) method
Result:
Asx: 0.99 (1)
Glx: 0.97 (1)
Ser: 0.94 (1)
*Ala: 1.00 (1)
Ile: 0.99 (1)
Trp: 0.78 (1)
*Ala was used as a standard amino acid. The values in parentheses indicate calculated values.

Example 22

Synthesis of HCNP(3-9)$NH_2$:

To 0.71 g of HCNP(3-9)$NH_2$ peptide resin described in Example 9 were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 100 ml of 10% acetic acid aqueous solution. The solution was applied to reverse phase YMC-A363(S-5)-ODS column (30 $\phi$ x 250 mm) previously equilibrated with 0.1% TFA aqueous solution. After the column was washed with 0.1% TFA aqueous solution, the peptide was eluted with linear gradient of 0 to 21% acetonitrile for 180 minutes at a flow rate of 7.0 ml/min. The eluent was monitored at A280 nm. The fractions containing the desired product were collected and lyophilized to give 22.8 mg of HCNP(3-9)$NH_2$.

The thus obtained HCNP(3-9)$NH_2$ was eluted at retention time of 18.8 minutes with linear density gradient of 10-50% aqueous acetonitrile containing 0.1% TFA through reverse phase YMC-AM303(S-5)-ODS column (4.6 $\phi$ x 250 mm). The thus obtained peptide was identified by amino acid analysis as follows.
Amino acid analysis
Hydrolysis: 4N Methanesulfonic acid, 2% tryptamine, at 110° C for 24 hours

Analysis method: PICO-TAG (reverse phase-PTC amino acid) method
Result:
Asx: 0.99 (1)
Glx: 0.96 (1)
Ser: 0.92 (1)
Gly: 0.97 (1)
*Ala: 1.00 (1)
Ile: 0.99 (1)
Trp: 0.82 (1)
*Ala was used as a standard amino acid. The values in parentheses indicate calculated values.

Example 23

Synthesis of $HCNP(4\text{-}8)NH_2$:

To 1.16 g of $HCNP(4\text{-}8)NH_2$ peptide resin described in Example 10 were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 100 ml of 10% acetic acid aqueous solution. The solution was applied to reverse phase YMC-A363(S-5)-ODS column (30 φ x 250 mm) previously equilibrated with 0.1% aqueous TFA solution. After the column was washed with 0.1% TFA aqueous solution, the peptide was eluted with linear gradient of 0 to 17% acetonitrile for 360 minutes at a flow rate of 7.0 ml/min. The eluent was monitored at A280 nm. The fractions containing the desired product were collected and lyophilized to give 121.3 mg of $HCNP(4\text{-}8)NH_2$.

The thus obtained $HCNP(4\text{-}8)NH_2$ was eluted at retention time of 16.2 minutes with linear density gradient of 10-50% aqueous acetonitrile containing 0.1% TFA through reverse phase YMC-AM303(S-5)-ODS column (4.6 φ x 250 mm). The thus obtained peptide was identified by amino acid analysis as follows.
Amino acid analysis
Hydrolysis: 4N Methanesulfonic acid, 2% tryptamine, at 110°C for 24 hours
Analysis method: PICO-TAG (reverse phase-PTC amino acid) method
Result:
Glx: 0.99 (1)
Ser: 0.91 (1)
*Ala: 1.00 (1)
Ile: 0.99 (1)
Trp: 0.71 (1)
*Ala was used as a standard amino acid. The values in parentheses indicate calculated values.

Example 24

Synthesis of HCNP(4-8):

Chloromethylated polystyrene vinylbenzene resin (1% divinylbenzene with an initial chloride loading of 0.68 mmol/g of the resin) of 100 to 200 mesh was employed.

Upon synthesis of HCNP(4-8), 2.31 g of Boc-Ala-OH was dissolved in a mixture of 10 ml of ethyl alcohol and 3 ml of water and, pH was adjusted to 7 with 20% cesium carbonate aqueous solution. The solution was concentrated in vacuo and desicated. To the residue were added 120 ml of DMF and then 15 g of the chloromethylated resin. The mixture was stirred at 50°C for 12 hours and then at room temperature for further 12 hours to esterify. The resulting Boc-Ala-O-resin was filtered and washed successively with DMF, 90% DMF, DMF and ethyl alcohol and then desicated. Yield, 15.8 g.

Three grams of this Boc-Ala-O-resin were charged in a solid phase synthesis reactor. Following

Schedule 2 described in Example 10, Boc-Trp-OH, Boc-Gln-OH, Boc-Ser(Bzl)-OH and Boc-Ile-OH were successively coupled with the resin. Thus, 4.01 g of HCNP(4-8) peptide resin was obtained.

To 2.00 g of this HCNP(4-8) peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 200 ml of 2.5% acetic acid aqueous solution. The solution was applied to reverse phase YMC-ODS-(S-10/20) column (30 ϕ x 300 mm) previously equilibrated with 0.1% TFA aqueous solution. After the column was washed with 0.1% TFA aqueous solution, the peptide was eluted with linear gradient of 0 to 16% acetonitrile for 200 minutes at a flow rate of 6.0 ml/min. The eluent was monitored at A280 nm. The fractions containing the desired product were collected and lyophilized to give 166.3 mg of HCNP(4-8).

The thus obtained HCNP(4-8) was eluted at retention time of 17.9 minutes with linear density gradient of 10-50% aqueous acetonitrile containing 0.1% TFA through reverse phase YMC-AM303(S-5)-ODS column (4.6 ϕ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Glx: 1.03 (1)
Ser: 0.97 (1)
*Ala: 1.00 (1)
Ile: 1.11 (1)
Trp: 0.69 (1)
*Ala was used as a standard amino acid. The values in parentheses indicate calculated values.

Example 25

Synthesis of $NH_2(CH)_5CO$-HCNP(2-7):

Upon synthesis of $NH_2(CH_2)_5CO$-HCNP(2-7), 13.1 g of $NH_2(CH_2)_5COOH$ was dissolved in a mixture of 50 ml of THF and 50 ml of water. Under ice cooling, 14 ml of TEA was added and 24.0 g of $(Boc)_2O$ was dropwise added to the solution followed by stirring at room temperature for 12 hours. To the reaction solution were added 100 ml of water and 100 ml of AcOEt. After separation, the aqueous phase was rendered acidic with citric acid followed by extracting twice with 100 ml of AcOEt. After washing with saturated sodium chloride aqueous solution, the organic phase was dried over magnesium sulfate and the solvent was distilled off. The residue was dissolved in 400 ml of diethyl ether and 9.9 g of CHA was dropwise added to the solution followed by stirring for 30 minutes. The crystals were taken out by filtration, washed with diethyl ether and desicated to give 28.3 g of Boc-$NH(CH_2)_5COOH$•CHA.

In a solid phase synthesis reactor, 0.50 g of HCNP(2-7) peptide resin described in Example 11 was charged and coupled according to Schedule 2 described in Example 10, using Boc-$NH(CH_2)_5COOH$•CHA described above, instead of the amino group-protected amino acid. In this case, Boc-$NH(CH_2)_5COOH$•CHA was used after the CHA salt was removed, prior to the coupling, by suspending a necessary amount of Boc-$NH(CH_2)_5COOH$•CHA in a mixture of AcOEt and 10% citric acid to fully dissolve it, washing the organic phase further with saturated sodium chloride aqueous solution, drying over magnesium sulfate and distilling off the solvent. Thus, 0.51 g of $NH_2(CH_2)_5CO$-HCNP(2-7) peptide resin was obtained.

To 0.51 g of this $NH_2(CH_2)_5CO$-HCNP(2-7) peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 200 ml of 2.5% acetic acid aqueous solution. The solution was applied to reverse phase YMC-ODS(S-10/20) column (30 ϕ x 300 mm) previously

equilibrated with 0.1% TFA aqueous solution. After the column was washed with 0.1% TFA aqueous solution, the peptide was eluted with linear gradient of 0 to 21% acetonitrile for 200 minutes at a flow rate of 6.0 ml/min. The eluent was monitored at A280 nm. The fractions containing the desired product were collected and lyophilized to give 60.9 mg of $NH_2(CH_2)_5CO$-HCNP(2-7) as the primary purification product. The primarily purified $NH_2(CH_2)_5CO$-HCNP(2-7) was dissolved in 100 ml of 5% acetic acid aqueous solution. The solution was applied to reverse phase YMC-SH-343-5(S-5) ODS column (20 $\phi$ x 500 mm) previously equilibrated with 0.1% TFA aqueous solution. After the column was washed with 0.1% TFA aqueous solution, the peptide was eluted with linear gradient of 0 to 19% acetonitrile for 180 minutes, then to 22% for 60 minutes and further to 24% for 60 minutes at a flow rate of 7.0 ml/min. The eluent was monitored at A280 nm. The fractions containing the desired product were collected and lyophilized to give 34.3 mg of $NH_2(CH_2)_5CO$-HCNP(2-7).

The thus obtained $NH_2(CH_2)_5CO$-HCNP(2-7) was eluted at retention time of 22.1 minutes with linear density gradient of 10-50% aqueous acetonitrile containing 0.1% TFA through reverse phase YMC-AM303-(S-5)-ODS column (4.6 $\phi$ x 250 mm). The thus obtained peptide was identified by amino acid analysis as follows.

Amino acid analysis

Hydrolysis: 4N Methanesulfonic acid, 2% tryptamine, at 110° C for 24 hours

Analysis method: PICO-TAG (reverse phase-PTC amino acid) method

Result:

Asx: 0.94 (1)

Glx: 0.91 (1)

Ser: 0.90 (1)

*Ala: 1.00 (1)

Ile: 1.02 (1)

Trp: 0.62 (1)

6-Aminohexanoic acid: 0.72 (1)

*Ala was used as a standard amino acid. The values in parentheses indicate calculated values.

## Example 26

Synthesis of $HOOC(CH_2)_3CO$-HCNP(2-7):

In a solid phase synthesis reactor, 0.50 g of HCNP(2-7) peptide resin described in Example 11 was charged. According to Schedule 2 described in Example 10, deprotection and neutralization at the steps 1 to 9 were carried out. At the step 10, 15 ml of a solution of 0.34 g of glutaric anhydride in methylene chloride was added to react. The steps 11 to 15 were carried out and 15 ml of a solution of 0.34 g of glutaric anhydride and 0.84 ml of triethylamine in methylene chloride was again added to the system to react. Subsequently, washings at the steps 11, 12, 15 and 18 were carried out. After drying 0.46 g of $HOOC(CH_2)_3CO$-HCNP(2-7) peptide resin was obtained.

To 0.46 g of this $HOOC(CH_2)_3CO$-HCNP(2-7) peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 200 ml of 2.5% acetic acid aqueous solution. The solution was applied to reverse phase YMC-SH-343-5(S-5)-ODS column (20 $\phi$ x 250 mm) previously equilibrated with 0.1% TFA aqueous solution. After the column was washed with 0.1% TFA aqueous solution, the peptide was eluted with linear gradient of 0 to 25% acetonitrile for 180 minutes, further to 28% for 60 minutes and then to 30% for 60 minutes at a flow rate of 7.0 ml/min. The eluent was monitored at A280 nm. The fractions containing the desired product were collected and lyophilized to give 17.0 mg of $HOOC(CH_2)_3CO$-HCNP(2-7).

The thus obtained $HOOC(CH_2)_3CO$-HCNP(2-7) was eluted at retention time of 24.8 minutes with linear density gradient of 10-50% aqueous acetonitrile containing 0.1% TFA through reverse phase YMC-AM303-(S-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110 °C for 24 hours
Analysis method: PICO-TAG (reverse phase-PTC amino acid) method
Result:
Asx: 1.01 (1)
Glx: 0.99 (1)
Ser: 0.98 (1)
*Ala: 1.00 (1)
Ile: 1.08 (1)
Trp: 0.67 (1)
*Ala was used as a standard amino acid. The values in parentheses indicate calculated values.

Example 27

Synthesis of $NH_2C(=NH)NHCH_2CO$-HCNP(2-7):

Upon synthesis of $NH_2C(=NH)NHCH_2CO$-HCNP(2-7), 10.0 g of guanidoacetic acid was dissolved in 300 ml of 1 N hydrochloric acid. After stirring, the solution was concentrated in vacuo. Diethyl ether was added to the residue to crystalize. The crystals were taken out by filtration, washed with diethyl ether and desicated to give 13.1 g of guanidoacetate hydrochloride.

In a solid phase synthesis reactor, 0.50 g of HCNP(2-7) peptide resin described in Example 11 was charged and coupled, according to Schedule 2 described in Example 10, with guanidoacetate hydrochloride described above instead of the amino group-protected amino acid. In this case, DMF was used instead of 50% DMF-50% methylene chloride and no additive was added. The ninhydrin test was not performed but the coupling was carried out twice. After the first coupling reaction, neither the neutralization step nor the last acetylation step was carried out. Thus, 0.49 g of $NH_2C(=NH)NHCH_2CO$-HCNP(2-7) was obtained.

To 0.49 g of this $NH_2C(=NH)NHCH_2CO$-HCNP(2-7) peptide resin were added 3 ml of anisole, 0.5 ml of ethylmethyl sulfide and 20 ml of anhydrous hydrogen fluoride. The mixture was reacted at -20 °C for 60 minutes and then at 0 °C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 200 ml of 2.5% acetic acid aqueous solution. The solution was applied to reverse phase YMC-ODS(S-10/20) column (30 $\phi$ x 300 mm) previously equilibrated with 0.1% TFA aqueous solution. After the column was washed with 0.1% TFA aqueous solution, the peptide was eluted with linear gradient of 0 to 19% acetonitrile for 200 minutes at a flow rate of 6.0 ml/min. The eluent was monitored at A280 nm. The fractions containing the desired product were collected and lyophilized to give 7.2 mg of $NH_2C(=NH)NHCH_2CO$-HCNP(2-7).

The thus obtained $NH_2C(=NH)NHCN_2CO$-HCNP(2-7) was eluted at retention time of 21.5 minutes with linear density gradient of 10-50% aqueous acetonitrile containing 0.1% TFA through reverse phase YMC-AM303(S-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110 °C for 24 hours
Analysis method: PICO-TAG (reverse phase-PTC amino acid) method
Result:
Asx: 1.05 (1)
Glx: 1.02 (1)
Ser: 0.97 (1)
*Ala: 1.00 (1)
Ile: 1.08 (1)
Trp: 0.73 (1)
*Ala was used as a standard amino acid. The values in parentheses indicate calculated values.

Example 28

Synthesis of $HCNP(1-7)NH(CH_2)_5COOH$:

Chloromethylated polystyrene vinylbenzene resin (1% divinylbenzene with an initial chloride loading of 0.66 mmol/g of the resin) of 100 to 200 mesh was employed.

Upon synthesis of $HCNP(1-7)NH(CH_2)_5COOH$, 1.31 g of Boc-$NH(CH_2)_5COOH$•CHA was suspended in a mixture of AcOEt and 10% citric acid. After fully dissolved, the organic phase was washed with saturated sodium chloride aqueous solution, and dried over magnesium sulfate. The solvent was distilled off to remove the CHA salt. The residue was dissolved in a mixture of 10 ml of ethyl alcohol and 3 ml of water and, pH was adjusted to with 20% cesium carbonate aqueous solution. The solution was concentrated in vacuo and desicated. To the residue were added 40 ml of DMF and 5 g of the chloromethylated resin. The mixture was stirred at 50° C for 24 hours to esterify. The resulting Boc-$NH(CH_2)_5COO$-resin was filtered and washed successively with DMF, 90% DMF, DMF and ethyl alcohol and then desicated. Yield, 5.24 g.

In a solid phase synthesis reactor, 2.24 g of this Boc-$NH(CH_2)_5COO$-resin was successively coupled with Boc-Trp-OH, Boc-Gln-OH, Boc-Ser(Bzl)-OH, Boc-Ile-OH, Boc-Asp(OcHex)-OH and Boc-Ala-OH, following Schedule 2 described in Example 10. After the last coupling of Boc-Ala-OH and completion of the acetylation step, approximately 1/2 of the resin was withdrawn and the remaining resin was further coupled Boc-Ala-OH according to Schedule 2. Thus, 1.60 g of $HCNP(1-7)NH(CH_2)_5COOH$ peptide resin was obtained.

To 0.80 g of this $HCNP(1-7)NH(CH_2)_5COOH$ peptide resin were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 200 ml of 2.5% acetic acid aqueous solution. The solution was applied to reverse phase YMC-ODS(S-10/20) column (30 ϕ x 300 mm) previously equilibrated with 0.1% TFA aqueous solution. After the column was washed with 0.1% TFA aqueous solution, the peptide was eluted with linear gradient of 0 to 24% acetonitrile for 200 minutes at a flow rate of 6.0 ml/min. The eluent was monitored at A280 nm. The fractions containing the desired product were collected and lyophilized to give 6.67 mg of $HCNP(1-7)NH(CH_2)_5COOH$.

The thus obtained $HCNP(1-7)NH(CH_2)_5COOH$ was eluted at retention time of 25.7 minutes with linear density gradient of 10-50% aqueous acetonitrile containing 0.1% TFA through reverse phase YMC-AM3-3-(S-5)-ODS column (4.6 ϕ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Asx: 1.05 (1)
Glx: 1.03 (1)
Ser: 0.98 (1)
*Ala: 2.00 (2)
Ile: 1.10 (1)
Trp: 0.82 (1)
6-Aminohexanoic Acid: 0.72 (1)
*Ala was used as a standard amino acid. The values in parentheses indicate calculated values.

Example 29

Synthesis of $HCNP(2-7)NH(CH_2)_8NH_2$:

(1) Synthesis of HCl•$H_2N(CH_2)_8NH$-Z

In 1 liter of acetonitrile was dissolved 20.0 g of $H_2N(CH_2)_8NH_2$. Under ice cooling, 139 ml of 1 N sodium hydroxide aqueous solution was added to the solution and 22.2 ml of Z-Cl was dropwise added to

the mixture followed by stirring for 3 hours. After the reaction solution was neutralized with 1 N hydrochloric acid, the precipitated crystals were filtered. The filtrate was further concentrated in vacuo and acetonitrile was distilled off. The precipitated crystals were filtered, washed with water and desicated to give 7.52 g of $HCl \cdot H_2N(CH_2)_8NH$-Z.

(2) Synthesis of Boc-Trp-$NH(CH_2)_8NH$-Z

In 20 ml of DMF was suspended 1.0 g of $HCl \cdot H_2N(CH_2)_8NH$-Z and, 0.37 g of TEA was added to the suspension to neutralize and dissolve. To the solution were added 0.50 g of HOBt and 1.1 g of Boc-Trp-OH to dissolve. Under ice cooling, 0.70 g of EDC•HCl was added to the solution. The mixture was stirred for 30 minutes and then at room temperature for 5 hours. After 200 ml of AcOEt was added to the reaction solution, the mixture was washed successively with 5% citric acid aqueous solution, saturated sodium hydrogencarbonate aqueous solution and saturated sodium chloride aqueous solution. After drying over magnesium sulfate, the solvent was distilled off to give 1.94 g of Boc-Trp-$NH(CH_2)_8NH$-Z.

(3) Synthesis of Boc-Gln-Trp-$NH(CH_2)_8NH$-Z

In 20 ml of acetonitrile was dissolved 1.94 g of Boc-Trp-$NH(CH_2)_8NH$-Z and, 2.48 g of methanesulfonic acid was added to the solution under ice cooling. The temperature was elevated to room temperature. After stirring for an hour, 20 ml of DMF was added to the reaction solution and the resulting mixture was neutralized with 2.61 g of TEA under ice cooling. After 0.54 g of HOBt and 0.98 g of Boc-Gln-OH were dissolved in the solution, 0.76 g of EDC•HCl was added to the solution followed by stirring for 30 minutes and then at room temperature for 5 hours. To the reaction solution was added 200 ml of AcOEt. The mixture was washed successively with 5% citric acid aqueous solution, saturated sodium hydrogencarbonate aqueous solution and saturated sodium chloride aqueous solution. After drying over magnesium sulfate, the solvent was distilled off to give 2.05 g of Boc-Gln-Trp-$NH(CH_2)_8NH$-Z

(4) Synthesis of Boc-Ser(Bzl)-Gln-Trp-$NH(CH_2)_8NH$-Z

In 20 ml of acetonitrile was dissolved 2.05 g of Boc-Gln-Trp-$NH(CH_2)_8NH$-Z and, 3.00 g of methanesulfonic acid was added to the solution under ice cooling. The temperature was elevated to room temperature. After stirring for 3 hours, 20 ml of DMF was added to the reaction solution and the resulting mixture was neutralized with 3.16 g of TEA under ice cooling. After 0.46 g of HOBt and 1.01 g of Boc-Ser(Bzl)-OH were dissolved in the solution, 0.65 g of EDC•HCl was added to the solution followed by stirring for 30 minutes and then at room temperature for 2 hours. The reaction solution was dropwise added to 150 ml of saturated sodium chloride aqueous solution and the precipitated crystals were taken out by filtration. After washing three times with 50 ml of hexane, the residue was desicated to give 2.29 g of Boc-Ser(Bzl)-Gln-Trp-NH-$(CH_2)_8NH$-Z.

(5) Synthesis of Boc-Ile-Ser(Bzl)-Gln-Trp-$NH(CH_2)_8NH$-Z

In 20 ml of acetonitrile was dissolved 2.29 g of Boc-Ser(Bzl)-Gln-Trp-$NH(CH_2)_8NH$-Z and, 2.02 g of methanesulfonic acid was added to the solution under ice cooling. The temperature was elevated to room temperature. After stirring for an hour, 20 ml of DMF was added to the reaction solution and the resulting mixture was neutralized with 2.12 g of TEA under ice cooling. After 0.44 g of HOBt and 0.77 g of Boc-Ile-OH were dissolved in the solution, 10.61 g of EDC•HCl was added to the solution followed by stirring for 30 minutes and then at room temperature for 2 hours. The reaction solution was dropwise added to 150 ml of saturated sodium chloride aqueous solution and the precipitated crystals were taken out by filtration. After washing three times with 50 ml of hexane, the residue was desicated to give 2.38 g of Boc-Ile-Ser(Bzl)-Gln-Trp-$NH(CH_2)_8NH$-Z.

(6) Synthesis of Boc-Asp(OcHex)-Ile-Ser(Bzl)-Gln-Trp-$NH(CH_2)_8NH$-Z

In 20 ml of acetonitrile was dissolved 2.38 g of Boc-Ile-Ser(Bzl)-Gln-Trp-NH$(CH_2)_8$NH-Z and, 2.71 g of methanesulfonic acid was added to the solution under ice cooling. The temperature was elevated to room temperature. After stirring for 2 hours, 20 ml of DMF and 20 ml of NMP were added to the reaction solution and the resulting mixture was neutralized with 2.91 g of TEA under ice cooling. After 0.36 g of HOBt and 0.84 g of Boc-Asp(OcHex)-OH were dissolved in the solution, 0.51 g of EDC•HCl was added to the solution followed by stirring for 30 minutes and then at room temperature for 2 hours. The reaction solution was dropwise added to 150 ml of saturated sodium chloride aqueous solution and the precipitated crystals were taken out by filtration. After washing three times with 50 ml of hexane, the residue was desicated to give 2.80 g of Boc-Asp(OcHex)-Ile-Ser(Bzl)-Gln-Trp-NH$(CH_2)_8$NH-Z.

(7) Synthesis of Boc-Ala-Asp(OcHex)-Ile-Ser(Bzl)-Gln)-Trp-NH$(CH_2)_8$NH-Z

In 10 ml of acetonitrile was dissolved 0.60 g of Boc-Asp(OcHex)-Ile-Ser(Bzl)-Gln-Trp-NH$(CH_2)_8$NH-Z and, 0.74 g of methanesulfonic acid was added to the solution under ice cooling. The temperature was elevated to room temperature. After stirring for an hour, 20 ml of DMF and 15 ml of NMP were added to the reaction solution and the resulting mixture was neutralized with 0.78 g of TEA under ice cooling. After 0.08 g of HOBt and 0.11 g of Boc-Ala-OH were dissolved in the solution, 0.11 g of EDC•HCl was added to the solution followed by stirring for 30 minutes and then at room temperature for 3 hours. The reaction solution was dropwise added to 150 ml of saturated sodium chloride aqueous solution and the precipitated crystals were taken out by filtration. After washing three times with 50 ml of hexane, the residue was desicated to give 0.71 g of Boc-Ala-Asp(OcHex)-Ile-Ser(Bzl)-Gln-Trp-NH$(CH_2)_8$NH-Z.

(8) Synthesis of HCNP(2-7)NH$(CH_2)_8NH_2$

To 0.71 g of Boc-Ala-Asp(OcHex)-Ile-Ser(Bzl)-Gln-Trp-NH$(CH_2)_8$NH-Z were added 3 ml of anisole, 0.5 ml of ethylmethyl sulfide and 20 ml of anhydrous hydrogen fluoride. The mixture was reacted at -20° C for 60 minutes and then at 0° C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 50 ml of 10% acetic acid aqueous solution. The solution was applied to reverse phase YMC-SH-343-5(S-5) ODS column (20 $\phi$ x 250 mm) previously equilibrated with 0.1% TFA aqueous solution. After the column was washed with 0.1% TFA aqueous solution, the peptide was eluted with linear gradient of 0 to 23% acetonitrile for 180 minutes, then to 26% for 60 minutes and further to 28% for 60 minutes at a flow rate of 7.0 ml/min. The eluent was monitored at A280 nm. The fractions containing the desired product were collected and lyophilized to give 170.7 mg of HCNP(2-7)NH$(CH_2)_8NH_2$.

The thus obtained HCNP(2-7)NH$(CH_2)_8NH_2$ was eluted at retention time of 23.4 minutes with linear density gradient of 10-50% aqueous acetonitrile containing 0.1% TFA through reverse phase YMC-AM303-(S-5)-ODS column (4.6 $\phi$ x 250 mm). The thus obtained peptide was identified by amino acid analysis as follows.

Amino acid analysis
Hydrolysis: 4N Methanesulfonic acid, 2% tryptamine, at 110° C for 24 hours
Analysis method: PICO-TAG (reverse phase-PTC amino acid) method
Result:
Asx: 0.80 (1)
Glx: 0.79 (1)
Ser: 0.89 (1)
*Ala: 1.00 (1)
Ile: 1.01 (1)
Trp: 0.65 (1)
*Ala was used as a standard amino acid. The values in parentheses indicate calculated values.

Example 30

Synthesis of $HCNP(2\text{-}7)NH(CH_2)_4NHC(=NH)NH_2$:

(1) Synthesis of Boc-$NH(CH_2)_4NHC(=NH)NH$-Tos

In 10 ml of water was dissolved 0.55 g of agmatine sulfate and, 0.36 g of TEA was added to the solution to neutralize. After 0.78 g of $(Boc)_2O$ and 2 ml of dioxane were added to the solution, the resulting mixture was stirred at room temperature for 24 hours. Water was added to the reaction solution. The mixture was washed with diethyl ether to give Boc-$NH(CH_2)_4NHC(=NH)NH_2$ aqueous solution (ca. 30 ml). Under ice cooling, 1.2 g of NaOH was added to the aqueous solution and a solution of 2.75 g of Tos-Cl in 10 ml of THF was dropwise added thereto followed by stirring at room temperature for 15 hours. After the reaction solution was neutralized with 4 N HCl, AcOEt was added to the system. After washing with saturated sodium chloride aqueous solution and drying over magnesium sulfate, the solvent was distilled off to give 1.22 g of Boc-$NH(CH_2)_4NHC(=NH)NH$-Tos.

(2) Synthesis of Boc-Trp-$NH(CH_2)_4NHC(=NH)NH$-Tos

In 10 ml of acetonitrile was dissolved 1.22 g of Boc-$NH(CH_2)_4NHC(=NH)NH$-Tos and 0.91 g of methanesulfonic acid was added to the solution. The mixture was stirred at room temperature for 30 minutes. The reaction solution was neutralized with 0.90 g of TEA under ice cooling. After 20 ml of DMF, successively 0.51 g of HOBt and then 1.16 g of Boc-Trp-OH were then added to the system to dissolve them, 0.73 g of EDC•HCl was added to the solution followed by stirring at room temperature for 3 hours. After acetonitrile was distilled off in vacuo, AcOEt and water were added to the residue. The mixture was washed successively with 10% citric acid aqueous solution, saturated sodium hydrogencarbonate aqueous solution and saturated sodium chloride aqueous solution. After drying over magnesium sulfate, the solvent was distilled off to give 2.09 g of Boc-Trp-$NH(CH_2)_4NHC$-$(=NH)NH$-Tos.

(3) Synthesis of Boc-Gln-Trp-$NH(CH_2)_4NHC(=NH)NH$-Tos

In 20 ml of acetonitrile was dissolved 2.09 g of Boc-Trp-$NH(CH_2)_4NHC(=NH)NH$-Tos and, 3.53 g of methanesulfonic acid was added to the solution. The mixture was stirred at room temperature for 2 hr. Under ice cooling, the reaction solution was neutralized with 3.70 g of TEA. After 20 ml of DMF, 0.59 g of HOBt and then 1.09 g of Boc-Gln-OH were successively added to the system to dissolve them, 0.84 g of EDC.HCl was added to the solution followed by stirring at room temperature for 2.5 hours. After acetonitrile was distilled off in vacuo, AcOEt and water were added to the residue. The mixture was washed successively with 10% citric acid aqueous solution, saturated sodium hydrogencarbonate aqueous solution and saturated sodium chloride aqueous solution. After drying over magnesium sulfate, the solvent was distilled off to give 2.27 g of Boc-Gln-Trp-$NH(CH_2)_4NHC(=NH)NH$-Tos.

(4) Synthesis of Boc-Ser(Bzl)-Gln-Trp-$NH(CH_2)_4NHC(=NH)NH$-Tos

In 20 ml of acetonitrile was dissolved 2.27 g of Boc-Gln-Trp-$NH(CH_2)_4NHC(=NH)NH$-Tos and, 3.12 g of methanesulfonic acid was added to the solution under ice cooling. The mixture was stirred at room temperature for 30 minutes. The reaction solution was neutralized with a solution of 3.70 g of TEA in 15 ml of acetonitrile under ice cooling. After 50 ml of DMF was added and 0.52 g of HOBt and 8.05 g of Boc-Ser-(Bzl)-OH were then added to the system to dissolve them, 5.23 g of EDC•HCl was added to the solution followed by stirring at room temperature overnight. AcOEt and water were added to the reaction solution. The mixture was washed successively with 10% citric acid aqueous solution, saturated sodium hydrogencarbonate aqueous solution and saturated sodium chloride aqueous solution. After drying over magnesium sulfate, the solvent was distilled off. The residue was recrystallized from AcOEt-diethyl ether. The crystals were collected by filtration, washed with diethyl ether and then desicated to give 1.96 g of Boc-Ser(Bzl)-Gln-Trp-$NH(CH_2)_4NHC(=NH)NH$-Tos.

(5) Synthesis of Boc-Ile-Ser(Bzl)-Gln-Trp-$NH(CH_2)_4NHC(=NH)NH$-Tos

In 50 ml of acetonitrile was dissolved 1.96 g of Boc-Ser(Bzl)-Gln-Trp-NH$(CH_2)_4$NHC(=NH)NH-Tos and, a solution of 2.15 g of methanesulfonic acid in 10 ml of acetonitrile was added to the solution. The mixture was stirred at room temperature for an hour. The reaction solution was neutralized with a solution of 2.26 g of TEA in 25 ml of acetonitrile under ice cooling. After 50 ml of DMF was added and 0.60 g of HOBt and 1.08 g of Boc-Ile-OH were then added to the mixture to dissolve them, 0.85 g of EDC•HCl was added to the solution followed by stirring at room temperature for overnight. AcOEt and water were added to the reaction solution. The mixture was washed successively with 10% citric acid aqueous solution, saturated sodium hydrogencarbonate aqueous solution and saturated sodium chloride aqueous solution. Hexane was added to the organic phase and the precipitated crystals were collected by filtration. After washing with hexane, the crystals were desicated to give 1.79 g of Boc-Ile-Ser(Bzl)-Gln-Trp-NH$(CH_2)_4$NHC(=NH)NH-Tos.

(6) Synthesis of Boc-Asp(OcHex)-Ile-Ser(Bzl)-Gln-Trp-NH$(CH_2)_4$NHC(=NH)NH-Tos

In 60 ml of acetonitrile was dissolved 1.79 g of Boc-Ile-Ser(Bzl)-Gln-Trp-NH$(CH_2)_4$NHC(=NH)NH-Tos and, a solution of 1.74 g of methanesulfonic acid in 10 ml of acetonitrile was added to the solution. The mixture was stirred at room temperature for 1.5 hour. The reaction solution was neutralized with a solution of 1.83 g of TEA in 30 ml of acetonitrile under ice cooling. After 100 ml of DMF was added and 0.48 g of HOBt, 1.14 g of Boc-Asp(OcHex)-OH were added to the mixture to dissolve them. Then, 0.69 g of EDC•HCl was added to the solution followed by stirring at room temperature for 2 hours. Under ice cooling, 200 ml of saturated sodium chloride aqueous solution was dropwise added to the reaction solution. The precipitated crystals were collected by filtration. After washing with water, the crystals were desicated to give 2.86 g of Boc-Asp(OcHex)-Ile-Ser(Bzl)-Gln-Trp-NH$(CH_2)_4$NHC-(=NH)NH-Tos.

(7) Synthesis of Boc-Ala-Asp(OcHex)-Ile-Ser(Bzl)-Gln-Trp-NH$(CH_2)_4$NHC(=NH)NH-Tos

In 40 ml of acetonitrile was dissolved 2.86 g of Boc-Asp(OcHex)-Ile-Ser(Bzl)-Gln-Trp-NH$(CH_2)_4$NHC-(=NH)NH-Tos and, a solution of 2.32 g of methanesulfonic acid in 10 ml of acetonitrile was added to the solution. The mixture was stirred at room temperature for an hour. The reaction solution was neutralized with a solution of 2.43 g of TEA in 25 ml of acetonitrile under ice cooling. After 100 ml of DMF was added and 0.65 g of HOBt, 0.91 g of Boc-Ala-OH were added to the mixture to dissolve them. Then 0.92 g of EDC•HCl was added to the solution followed by stirring at room temperature overnight. Under ice cooling, 200 ml of saturated sodium chloride aqueous solution was dropwise added to the reaction solution. The precipitates were collected by filtration. After washing with water, the precipitates were desicated to give 1.82 g of Boc-Ala-Asp(OcHex)-Ile-Ser(Bzl)-Gln-Trp-NH$(CH_2)_4$NHC(=NH)NH-Tos.

(8) Synthesis of HCNP(2-7)NH$(CH_2)_4$NHC(=NH)$NH_2$

To 0.91 g of Boc-Ala-Asp(OcHex)-Ile-Ser(Bzl)- Gln-Trp-NH$(CH_2)_4$NHC(=NH)NH-Tos were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 100 ml of 10% acetic acid aqueous solution. The solution was applied to reverse phase YMC-SH-343-5(S-5) ODS column (20 ϕ x 500 mm) previously equilibrated with 0.1% TFA. After the column was washed with 0.1% TFA aqueous solution, the peptide was eluted with linear gradient of 0 to 18% acetonitrile for 180 minutes, then to 21% for 60 minutes and further to 23% for 60 minutes at a flow rate of 7.0 ml/min. The eluent was monitored at A280 nm. The fractions containing the desired product were collected and lyophilized to give 122.4 mg of HCNP(2-7)NH$(CH_2)_4$NHC(=NH)$NH_2$.

The thus obtained HCNP(2-7)NH$(CH_2)_4$NHC-(=NH)$NH_2$ was eluted at retention time of 19.3 minutes with linear density gradient of 10-50% aqueous acetonitrile containing 0.1% TFA through reverse phase YMC-AM303(S-5)-ODS column (4.6 ϕ x 250 mm). The thus obtained peptide was identified by amino acid analysis as follows.
Amino acid analysis

Hydrolysis: 4N Methanesulfonic acid, 2% tryptamine, at 110 °C for 24 hours
Analysis method: PICO-TAG (reverse phase-PTC amino acid) method
Result:
Asx: 0.90 (1)
Glx: 0.86 (1)
Ser: 0.95 (1)
*Ala: 1.00 (1)
Ile: 1.00 (1)
Trp: 0.66 (1)
Agmatine: 1.18 (1)
*Ala was used as a standard amino acid. The values in parentheses indicate calculated values.

Example 31

Assay for biological activity:

The peptides were assayed for a biological activity in accordance with the method of Ojika et al. [Japanese Journal of Psycopharmacology, 7, 447-451 (1987)]. Namely, medial septum nucleous removed from rat embryonal brain (16 day embryo) is cut to small pieces, then the pieces were cultured in the Bottenstein's modified $N_2$ medium containing 1% FCS of saturation concentration, in Falcon's 35 mm plastic culture dish with 7% $CO_2$ mixed air, at 36 °C. The neurotrophic peptide or its derivative was added to the cultures at initial day. In a control group, no peptide is added. The neurotrophic peptide may be added to the culture at 3 days after the initial day. After 6 days culture, an acetylcholine synthesis activity of cultured tissue was assayed to determine the biological activity of the peptide. The acetylcholine synthesis activity was assayed as follows.

A cultured tissue was pre-incubated in high potasium Tyrode solution buffered with tris-HCl (pH 7.4) containing 98 mM of Nacl and 55 mM of Kcl , then incubated in a buffered solution containing 100 nM [$^3$H] choline chloride (15 Ci/mmol) at 37 °C for 30 minutes. After washing out of free [$^3$H] choline, the tissue was dissolved in 1 N formic acid/acetone (15 : 85) solution. Free [$^3$H] choline was converted to phosphocholine by choline kinase (0.1 unit/ml) and [$^3$H] acetylcholine was extracted with tetraphenyl boron (5 mg/ml acetonitrile) to determine the acetylcholine synthesis activity. The activity of cultured tissue for synthesis of acetylcholine was defined as an amount of synthesized acetylcholine per piece of cultured tissue.

(i) Biological activity of "HCNP" obtained in Example 1 is shown in Table 1.

At the concentration of 300 pg/ml "HCNP", the cultured tissue exhibited 202% acetylcholine synthesis activity as compared with control culture.

Thus, the biological activity of "HCNP" was confirmed.

Table 1:

| Enhancing effect on acetylcholine (Ach) synthesis in culture of rat medial septum nucleous (average value in triplet assay). | |
|---|---|
| Specimen | Ach synthesis activity (fmol/specimen) |
| Control | 4.2 ± 0.7 |
| "HCNP" | 8.5 ± 0.6 |

(ii) Ac-HCNP obtained Example 5 was assayed and the result was shown in Table 2.

At the concentration of 300 pg/ml Ac-HCNP, the cultured tissue exhibited 159% acetylcholine synthesis activity as compared with control culture.

Table 2:

| Enhancing effect on acetylcholine (Ach) synthesis in culture of rat medial septum nucleous (average value in triplet assay). | |
|---|---|
| Specimen | Ach synthesis activity (fmol/specimen) |
| Control | 6.8 ± 2.4 |
| Ac-HCNP | 10.8 ± 1.9 |

(iii) f-HCNP obtained in Example 6 was assayed, and the result was shown in Table 3.

At the concentration of 2.5 pg/ml f-HCNP, the cultured tissue exhibited 159% acetylcholine synthesis activity as compared with control culture.

Table 3:

| Enhancing effect on acetylcholine (Ach) synthesis in culture of rat medial septum nucleous (average value in triplet assay). | |
|---|---|
| Specimen | Ach synthesis activity (fmol/specimen) |
| Control | 7.1 ± 1.1 |
| f-HCNP | 11.3 ± 0.8 |

(iv) Some typical examples of neurotrophic peptide of the present invention was assayed and the specific biological activity was shown in Table 4.

In Table 4, the Ach synthesis activity is defined assuming control value as 100%.

Table 4:

| Enhancing effect on acetylcholine (Ach) synthesis in culture of rat media ceptum nuclei . | | |
|---|---|---|
| Compound | Concentration (ml/l) | Ash synthesis (%) |
| Ac-HCNP(1-9)$NH_2$ | $1 \times 10^{-8}$ | 227, 216 |
| HCNP(1-7)OH | $1 \times 10^{-8}$ | 274, 146, 75 |
| HCNP(4-11)OH | $1 \times 10^{-8}$ | 160, 139 |
| HCNP(2-7)OH | $1 \times 10^{-8}$ | 189, 174, 117, 168, 84 |
| HCNP(3-9)$NH_2$ | $1 \times 10^{-8}$ | 224, 138 |
| HCNP(4-8)$NH_2$ | $1 \times 10^{-8}$ | 324, 298, 143, 65 |

## Claims

1. A novel peptide comprising the whole or partial sequence of an amino acid sequence of formula (1):
Ala-Ala-Asp-Ile-Ser-Gln-Trp-Ala-Gly-Pro-Leu (1)
$NH_2$- and/or COOH-terminal-modified derivative of the peptide, or pharmaceutically acceptable salt thereof.

2. A novel peptide, $NH_2$- and/or COOH-terminal-modified derivative of the peptide or pharmaceutically acceptable salt thereof according to Claim 1, which comprises at least -Trp- of the formula (1).

3. A novel peptide, $NH_2$- and/or COOH-terminal-modified derivative of the peptide, or pharmaceutically acceptable salt thereof according to Claim 1, which comprises at least 3 consecutive amino acid residues of the formula (1).

4. A novel peptide, $NH_2$- and/or COOH-terminal-modified derivative of the peptide or pharmaceutically acceptable salt thereof according to Claim 1, which is represented by formula (II)
X-Z-Y (II)
wherein X is a hydrogen atom;

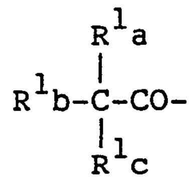

wherein $R^1a$ is a hydrogen atom, an unsubstituted or substituted alkyl group, amino, mono- or di-$C_1$-$C_4$ alkylamino, hydroxy, $-CO_2H$, guanidino, $C_1$-$C_4$ alkylguanidino, aryl, $C_1$-$C_4$ alkoxy, halo, $N(R^2)_3A$ (wherein $R^2$ is $C_1$-$C_4$ alkyl, and A is a counter ion selected from the group consisting of monovalent anions), $-CONR^3R^4$ (wherein each of $R^3$ and $R^4$ independently represents a hydrogen atom or a $C_1$-$C_4$ alkyl group) or a heterocyclic group, $R^1b$ is a hydrogen atom, an unsubstituted or substituted alkyl group or a halogen atom and $R^1c$ is a hydrogen atom, a $C_1$-$C_4$ alkyl group or a halogen atom;

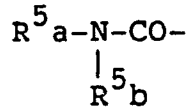

wherein $R^5a$ is a hydrogen atom, a $C_1$-$C_4$ alkyl group or an aryl group and $R^5b$ is a hydrogen atom or a $C_1$-$C_4$ alkyl group;
$R^6$-O-CO-
wherein $R^6$ is a $C_1$-$C_4$ alkyl group or an aryl group;
$R^7$-CO-
wherein $R^7$ is a hydrogen atom, an aryl group or a heterocyclic group; or
$R^8$-$SO_2$-
wherein $R^8$ is an unsubstituted or substituted alkyl group or an aryl group; Z is the whole or partial sequence of an amino acid sequence of the formula (1): and Y is

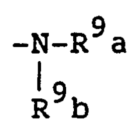

wherein $R^9a$ is a hydrogen atom, an unsubstituted or substituted alkyl group, hydroxy, aryl or a heterocyclic group; and $R^9b$ is a hydrogen atom or an unsubstituted or substituted alkyl group;
-O-$R^{10}$
wherein $R^{10}$ is an unsubstituted or substituted alkyl group, aryl or a heterocyclic group; or

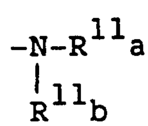

wherein $R^{11}a$ and $R^{11}b$ are combined together to form a nitrogen-containing saturated heterocyclic group.

5. A novel peptide, $NH_2$- and/or COOH-terminal modified derivative of the peptide, or pharmaceutically acceptable salt thereof according to Claim 4, wherein X is:

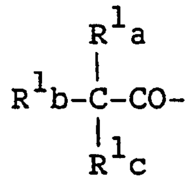

wherein $R^1a$ is a hydrogen atom, an unsubstituted or substituted alkyl group, amino, mono- or di-$C_1$-$C_4$ alkylamino, hydroxy, $-CO_2H$, guanidino, $C_1$-$C_4$ alkylguanidino, aryl, $C_1$-$C_4$ alkoxy, halo, $N(R^2)_3A$ (wherein $R^2$ is $C_1$-$C_4$ alkyl, and A is a counter ion selected from the group consisting of monovalent anions), $-CONR^3R^4$ (wherein each of $R^3$ and $R^4$ independently represents a hydrogen atom or a $C_1$-$C_4$ alkyl groups) or a heterocyclic group, $R^1b$ is a hydrogen atom, an unsubstituted or substituted alkyl group or a halogen atom and $R^1c$ is a hydrogen atom, a $C_1$-$C_4$ alkyl group or a halogen atom;

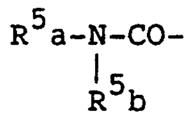

wherein $R^5a$ is a hydrogen atom, a $C_1$-$C_4$ alkyl group or an aryl group and $R^5b$ is a hydrogen atom or a $C_1$-$C_4$ alkyl group;

$R^6$-O-CO-

wherein $R^6$ is a $C_1$-$C_4$ alkyl group or an aryl group; or

$R^7$-CO-

wherein $R^7$ is a hydrogen atom, an aryl group or a heterocyclic group.

6. A novel peptide, $NH_2$- and/or COOH-terminal-modified derivative of the peptide, or pharmaceutically acceptable salt thereof according to Claim 4, wherein X is

$R^8$-$SO_2$-

wherein $R^8$ is an unsubstituted or substituted alkyl group or an aryl group.

7. A novel peptide, $NH_2$- and/or COOH-terminal-modified derivative of the peptide, or pharmaceutically acceptable salt thereof according to Claim 4, wherein Y is

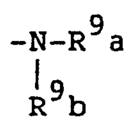

wherein $R^9a$ is a hydrogen atom, an unsubstituted or substituted alkyl group, hydroxy, aryl or a heterocyclic group; and $R^9b$ is a hydrogen atom or an unsubstituted or substituted alkyl group; or

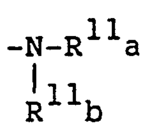

wherein $R^{11}a$ and $R^{11}b$ are combined together to form a nitrogen-containing saturated heterocyclic group.

8. A novel peptide, $NH_2$- and/or COOH-terminal-modified derivative of the peptide, or pharmaceutically acceptable salt thereof according to Claim 4, wherein Y is

-O-$R^{10}$

wherein is an unsubstituted or substituted alkyl group, aryl or a heterocyclic group.

9. A novel peptide, $NH_2$- and/or COOH-terminal-modified derivative of the peptide, or pharmaceutically acceptable salt thereof according to Claim 4, wherein Z is an amino acid sequence represented by general

formula:

$Z^1$-Trp-$Z^2$

wherein $Z^1$ represents Ala-Ala-Asp-Ile-Ser-Gln, Ala-Asp-Ile-Ser-Gln, Asp-Ile-Ser-Gln, Ile-Ser-Gln, Ser-Gln, Gln or a single bond; $Z^2$ represents Ala-Gly-Pro-Leu, Ala-Gly-Pro, Ala-Gly, Ala or a single bond; provided that when $Z^1$ represents a single bond, $Z^2$ cannot be a single bond.

10. A novel peptide or pharmaceutically acceptable salt thereof according to Claim 4, wherein X is a hydrogen atom and Y is a hydroxy group.

11. A novel peptide, $NH_2$- and/or COOH-terminal-modified derivative of the peptide, or pharmaceutically acceptable salt thereof according to Claim 4, wherein Z is Ala-Ala-Asp-Ile-Ser-Gln-Trp-Ala-Gly-Pro-Leu;
Ala-Ala-Asp-Ile-Ser-Gln-Trp-Ala-Gly-Pro;
Ala-Ala-Asp-Ile-Ser-Gln-Trp-Ala-Gly;
Ala-Ala-Asp-Ile-Ser-Gln-Trp-Ala;
Ala-Ala-Asp-Ile-Ser-Gln-Trp;
Ala-Asp-Ile-Ser-Gln-Trp-Ala-Gly-Pro-Leu;
Ile-Ser-Gln-Trp-Ala-Gly-Pro-Leu;
Gln-Trp-Ala-Gly-Pro-Leu;
Ala-Asp-Ile-Ser-Gln-Trp-Ala-Gly-Pro;
Ala-Asp-Ile-Ser-Gln-Trp-Ala;
Ala-Asp-Ile-Ser-Gln-Trp;
Asp-Ile-Ser-Gln-Trp-Ala-Gly; or
Ile-Ser-Gln-Trp-Ala.

12. A novel neurotrophic peptide derivable from a water soluble fraction of hippocampal tissue having a molecular weight of 700 to 1,400, as determined by gel filtration and having biological activity as an enhancer of acetylcholine synthesis in cholinergic neurons.

13. A novel neurotrophic peptide, or pharmaceutically acceptable salt thereof according to Claim 12, which has the following relative amino acid composition;

Asp: 1, Ile: 1, Ser: 1, Gln: 1, Trp: 1, Gly: 1, Pro: 1, Ala: 3, Leu: 1.

14. A novel peptide composed of the following amino acid sequence:

X-Ala-Asp-Ile-Ser-Gln-Trp-Ala-Gly-Pro-Leu

wherein X is Ala or acetylated Ala.

15. Ac-Ala-Ala-Asp-Ile-Ser-Gln-Trp-Ala-Gly-Pro-Leu- $NH_2$ or a pharmaceutically acceptable salt thereof.

16. Ac-Ala-Ala-Asp-Ile-Ser-Gln-Trp-Ala-Gly-$NH_2$ or a pharmaceutically acceptable salt thereof.

17. Ac-Ala-Ala-Asp-Ile-Ser-Gln-Trp-OH or a pharmaceutically acceptable salt thereof.

18. H-Ala-Ala-Asp-Ile-Ser-Gln-Trp-OH or a pharmaceutically acceptable salt thereof.

19. H-Ile-Ser-Gln-Trp-Ala-Gly-Pro-Leu-OH or a pharmaceutically acceptable salt thereof.

20. H-Gln-Trp-Ala-Gly-Pro-Leu-OH or a pharmaceutically aceptable salt thereof.

21. H-Ala-Asp-Ile-Ser-Gln-Trp-OH or a pharmaceutically acceptable salt thereof.

22. H-Asp-Ile-Ser-Gln-Trp-Ala-Gly-$NH_2$ or a pharmaceutically acceptable salt thereof.

23. H-Ile-Ser-Gln-Trp-Ala-$NH_2$ or a pharmaceutically acceptable salt thereof.

24. $NH_2(CH_2)_5CO$-Ala-Asp-Ile-Ser-Gln-Trp-OH or a pharmaceutically acceptable salt thereof.

25. $HOOC(CH_2)_3CO$-Ala-Asp-Ile-Ser-Gln-Trp-OH or a pharmaceutically acceptable salt thereof.

26. $NH_2C(=NH)NHCH_2CO$-Ala-Asp-Ile-Ser-Gln-Trp-OH or a pharmaceutically acceptable salt thereof.

27. H-Ala-Asp-Ile-Ser-Gln-Trp-$NH(CH_2)_4NHC(=NH)NH_2$ or a pharmaceutically acceptable salt thereof.

28. A method for preparing the novel peptide, or salt thereof of any one of Claims 12 to 14, which comprises the steps of:

(a) subjecting an extract solution from hippocampal tissue in mammals to reverse phase liquid chromatography, and

(b) collecting the desired product.

29. A method according to Claim 28, wherein a water soluble fraction of hippocampal tissue in mammals is subjected to gel filtration and then reverse phase liquid chromatography.

30. A method according to Claim 28, wherein the desired product is isolated and purified by monitoring the biological activity as an enhancer of acetylcholine synthesis in cholinergic neurons.

31. A method for preparing the novel peptide, derivative thereof or salt thereof of any one of Claims 1 to 11 and 14 to 27, which comprises chemical synthesis.

32. A method according to Claim 31, wherein the chemical synthesis is a conventional solid or liquid phase method.

33. A pharmaceutical composition for the treatment of neurological degenerative disorders which comprises at least one of the peptides, derivatives thereof and salts thereof of any one of Claims 1 to 27 as

an active ingredient.

34. A pharmaceutical composition for the treatment of dementia which comprises at least one of the peptides, derivatives thereof and salts thereof of any one of Claims 1 to 27 as an active ingredient.

35. A peptide according to any one of Claims 1 to 27 for use in a method for the treatment of the human or animal body by therapy.

36. Use of a peptide according to any one of Claims 1 to 27 in the manufacture of a medicament for use in a method for the treatment of the human or animal body as therapy for dementia.

FIG. 1

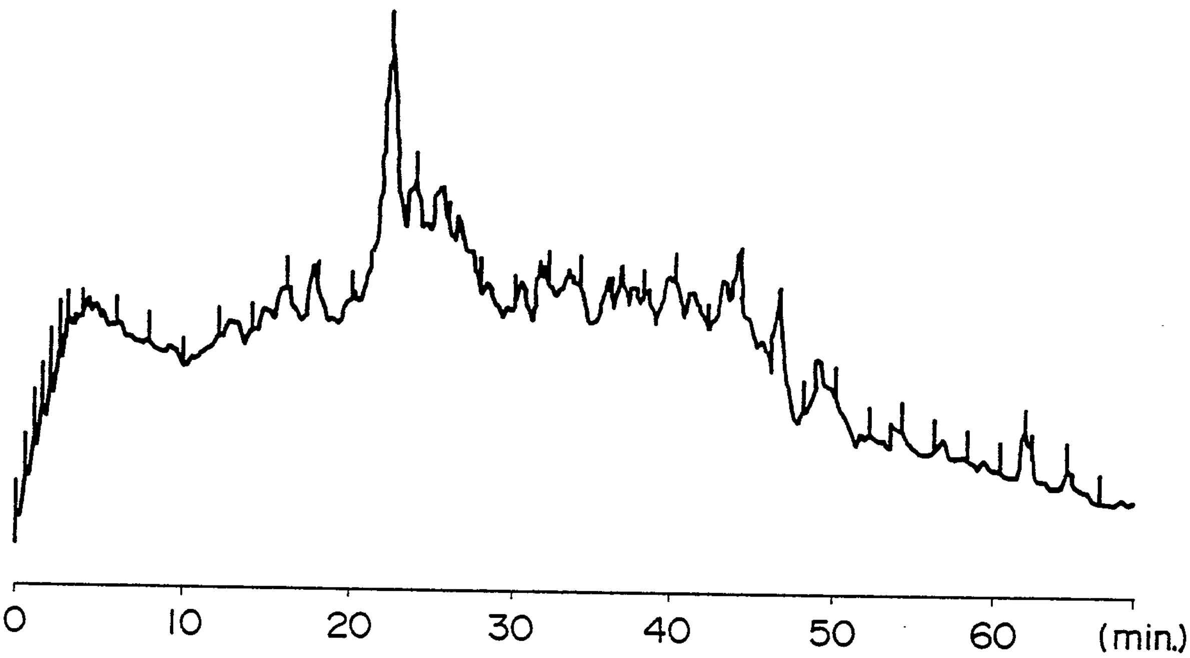


FIG. 2

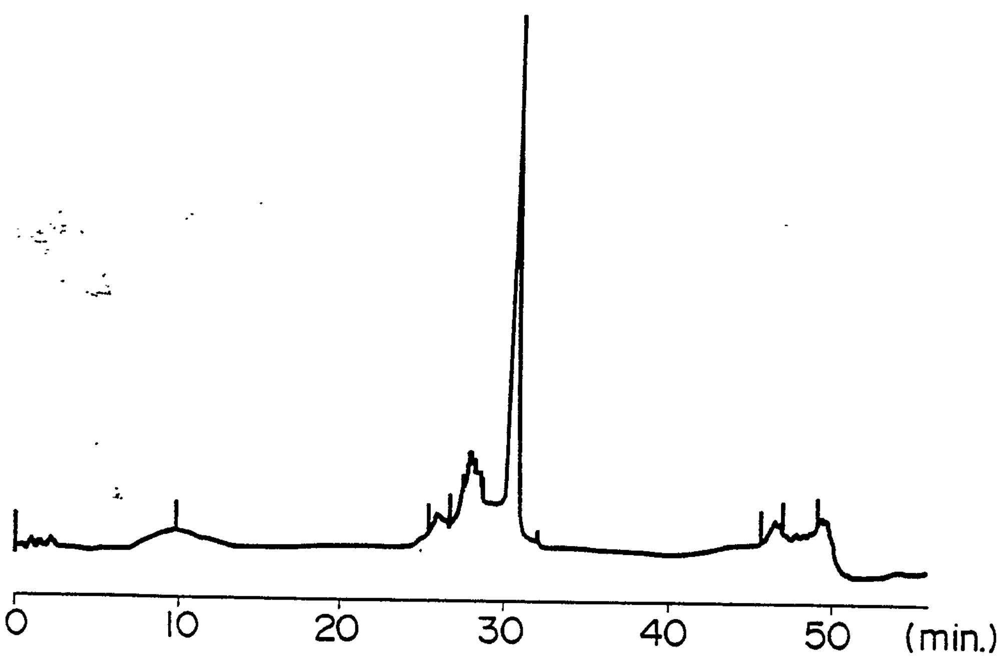

European Patent Office

# EUROPEAN SEARCH REPORT

Application Number

EP 90 30 3470

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 001 061 (SANDOZ AG)<br>* Claim 1 *<br>--- | 1-4,8-9 | C 07 K 7/06<br>C 07 K 5/04<br>A 61 K 37/02 |
| X | EP-A-0 134 986 (FARMITALIA CARLO ERBA S.p.A.)<br>* Claim 1 *<br>--- | 1-4,9 | |
| X | BIOCHEMICAL AND BIOPHYSICAL RESEARCH COMMUNICATIONS, vol. 119, no. 1, 29th February 1984, pages 14-20, Academic Press, Inc.; N. MINAMINO et al.: "Neuromedin C: A bombesin-like peptide identified in porcine spinal cord"<br>* Page 19, figure 5 *<br>--- | 1-4,9 | |
| A | FR-A-2 359 120 (SANDOZ S.A.)<br>* Claim 1 *<br>--- | 4-5 | |
| D,X | BRAIN RESEARCH, vol. 422, 1987, pages 92-98, Elsevier, Amsterdam, NL; J.R. BOSTWICK et al.: "Distinct influences of nerve growth factor and a central cholinergic trophic factor on medial septal explants"<br>* Abstract; page 93, last paragraph - page 94, first paragraph *<br>----- | 12 | TECHNICAL FIELDS SEARCHED (Int. Cl.5)<br>C 07 K |

The present search report has been drawn up for all claims

| Place of search | Date of completion of the search | Examiner |
|---|---|---|
| THE HAGUE | 12-07-1990 | BEVAN S.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document